(19) Europäisches Patentamt
European Patent Office
Office européen des brevets

(11) EP 1 644 086 B2

(12) # NEUE EUROPÄISCHE PATENTSCHRIFT

Nach dem Einspruchsverfahren

(45) Veröffentlichungstag und Bekanntmachung des Hinweises auf die Entscheidung über den Einspruch:
**12.09.2012 Patentblatt 2012/37**

(45) Hinweis auf die Patenterteilung:
**02.12.2009 Patentblatt 2009/49**

(21) Anmeldenummer: **04740940.4**

(22) Anmeldetag: **12.07.2004**

(51) Int Cl.:
***A61P 3/06*** (2006.01) ***A61P 3/10*** (2006.01)
***A61P 1/00*** (2006.01) ***A61P 9/00*** (2006.01)
***A61P 9/12*** (2006.01) ***A61K 35/20*** (2006.01)
***A61K 31/19*** (2006.01) ***A61K 31/7016*** (2006.01)

(86) Internationale Anmeldenummer:
**PCT/EP2004/007690**

(87) Internationale Veröffentlichungsnummer:
**WO 2005/004990 (20.01.2005 Gazette 2005/03)**

(54) **VERWENDUNG VON MOLKENPERMEAT ZUR BEHANDLUNG DES METABOLISCHEN SYNDROMS**

USE OF WHEY PERMEATE FOR THE TREATMENT OF METABOLIC SYNDROME

PERMEAT DE LACTOSERUM UTILISE POUR TRAITER LE SYNDROME METABOLIQUE

(84) Benannte Vertragsstaaten:
**AT BE BG CH CY CZ DE DK EE ES FI FR GB GR HU IE IT LI LU MC NL PL PT RO SE SI SK TR**

(30) Priorität: **10.07.2003 DE 10331202**

(43) Veröffentlichungstag der Anmeldung:
**12.04.2006 Patentblatt 2006/15**

(73) Patentinhaber: **"S.u.K." Beteiligungs GmbH
1010 Wien (AT)**

(72) Erfinder:
- **KRAUSKOPF, Jobst
29576 Barum (DE)**
- **SOWADA, Christian
2284 Norderstedt (DE)**

(74) Vertreter: **HOFFMANN EITLE
Patent- und Rechtsanwälte
Arabellastrasse 4
81925 München (DE)**

(56) Entgegenhaltungen:
**WO-A-03/011263**
**WO-A-03/105882**
**WO-A-2004/009070**
**WO-A1-2004//056207**
**WO-A2-01//37850**
**WO-A2-2005//000325**
**DE-A- 10 135 493**
**US-A- 5 639 501**
**US-A1- 2003 004 095**
**US-A1- 2003 118 662**
**US-B1- 6 399 140**

- **KAWASE M ET AL: "EFFECT OF ADMINISTRATION OF FERMENTED MILK CONTAINING WHEY PROTEIN CONCENTRATE TO RATS AND HEALTHY MEN ON SERUM LIPIDS AND BLOOD PRESSURE" JOURNAL OF DAIRY SCIENCE, AMERICAN DAIRY SCIENCE ASSOCIATION. CHAMPAIGN, ILLINOIS, US, Bd. 83, Nr. 2, Februar 2000 (2000-02), Seiten 255-263, XP000926924 ISSN: 0022-0302**

Bemerkungen:
Die Akte enthält technische Angaben, die nach dem Eingang der Anmeldung eingereicht wurden und die nicht in dieser Patentschrift enthalten sind.

EP 1 644 086 B2

## Beschreibung

### Technisches Gebiet

Die vorliegende Erfindung betrifft die Behandlung des Metabolischen Syndroms oder des Typ 2-Diabetes. Ferner wird die Verwendung von Molkenpermeat zur Herstellung diätetischer und pharmazeutischer Zusammensetzungen zur Prävention und Behandlung von Symptomen des Metabolischen Syndroms oder des Typ 2-Diabetes offenbart.

### Stand der Technik

Nach WHO-Kriterien sind 63 % aller Männer und 55 % aller Frauen in den industrialisierten Ländern übergewichtig. Ursache dieser bedenklichen Entwicklung ist die kalorische Überernährung mit Kohlenhydraten und Fetten bei weiterer Absenkung der täglichen Energieabgabe. Schon sehr kleine Differenzen in der täglichen Energiebilanz erzeugen über die Jahre einen großen kumulativen Effekt auf die Körpermasse. So führt 0,3 % Kalorienzufuhr pro Tag über dem Kalorienverbrauch zu einer Gewichtszunahme von 9.1 kg bei den 25 bis 55 Jahre alten Amerikanern. Die Übergewichtigen entwickeln häufig ein Metabolisches Syndrom (Reaven GM, Curr. Opin. Lipidol., 1997, 8 (1), S. 23-27).

Im Zusammenhang mit der Verwendung des Begriffs "Metabolisches Syndrom" sprechen Mediziner in der Regel vom sogenannten "tödlichen Quartett", das aus Übergewicht (Fettsucht), Bluthochdruck, erhöhtem Insulinspiegel und Fettstoffwechselstörung besteht. In der Fachliteratur wird das Metabolische Syndrom auch als 1. Frühsyndrom oder "Prädiabetes" bezeichnet (Pschyrembel, Klinisches Wörterbuch, 257 Aufl. (1994), S. 321).

Unter dem Begriff "Sydrom" versteht der Fachmann einen Symptomenkomplex; eine Gruppe von Krankheitszeichen (Symptomen), die für ein bestimmtes Krankheitsbild mit meist uneinheitlicher oder unbekannter Ätiologie bzw. Pathogenese charakteristisch sind.

Mit weiterer Verschlechterung des Metabolischen Syndroms entwickelt sich häufig ein Typ 2-Diabetes. Hervorstechendes Merkmal hierfür ist die Erhöhung des Blutzuckers. Des Weiteren begünstigen diese Erkrankungen ein erhöhtes Risiko von Folgeerkrankungen wie beispielsweise einer Arterienverkalkung. Weitere Gesundheitsrisiken die durch die pathologischen Veränderungen beim Diabetes bedingt sind, sind Augenleiden, Herz- und Gefäßerkrankungen, Schlaganfall, Herzinfarkt, Nierenerkrankungen etc.

In den vergangenen Jahrzehnten hat die Häufigkeit des Typ 2-Diabetes erheblich zugenommen. Das Statistische Bundesamt schätzt, dass derzeit rund vier Millionen Menschen in Deutschland betroffen sind. Andere Studien gehen jedoch von erheblich höheren Zahlen aus. Nach Schätzungen der Deutschen Diabetes Gesellschaft, wird 2006 jeder 10 Einwohner Deutschlands vom Typ 2-Diabetes betroffen sein. Bei übergewichtigen Diabetikern sind zumeist auch die Blutfettspiegel (Cholesterin, Triglyzeride) und der Blutdruck erhöht.

Im Gegensatz zum Typ 1-Diabetes wird beim Typ 2-Diabetes zwar Insulin produziert. Dieses kann vom Körper jedoch nicht mehr richtig verwertet werden. Das Insulin wird in der Bauchspeicheldrüse gebildet und sorgt dafür, dass Glucose aus der Nahrung in die Zellen gelangt. Wenn dem Körper durch die Nahrung Glucose zugeführt wird und der Blutzuckerspiegel im Blut ansteigt, wird vermehrt Insulin in das Blut abgegeben, um die Glucose in die Zellen zu befördern - der Blutzuckerspiegel sinkt dadurch wieder. Wenn der Körper das Insulin nicht mehr richtig auf das Insulin reagiert, werden die Zellen insulinresistent. Laut Roche Lexikon Medizin, 4. Auflage, Urban & Fischer Verlag, 1999, versteht man unter Insulinresistenz die starke Minderung oder das Ausbleiben der therapeutischen Insulinwirkung. Für das Entstehen einer Insulinresistenz gibt es drei Erklärungen. Erstens können IgG-Antikörper die biologische Wirksamkeit des Insulins hemmen und dadurch den Bedarf auf über 100 IE (Internationale Einheiten) täglich erhöhen. Zweitens kann es zu einer erhöhten enzymatischen Insulinspaltung kommen, oder drittens die Bindung des Insulins an seine Rezeptoren herabgesetzt sein. Als Folge gelangt nicht mehr ausreichend Energie in die Zellen, während der Blutzuckerspiegel hoch bleibt. Das führt dazu, dass die Bauchspeicheldrüse noch mehr Insulin ausschüttet, um den Blutzuckerspiegel zu senken. Durch die ständige Überproduktion von Insulin kommt es zu einer Erschöpfung der Insulin-produzierenden Beta-Zellen in der Bauchspeicheldrüse und schließlich zum Insulinmangel.

Heutzutage werden Typ 2-Diabetiker häufig mit oralen Antidiabetika behandelt. Hierzu zählen:

a) Medikamente, die die Aufnahme von Kohlenhydraten verzögern, bspw. Alpha-Glukosidasehemmer,

b) die Biguanide - eine Substanzgruppe, die sowohl die Zuckerresorption verzögern als auch die Zuckerneubildung in der Leber hemmen. Außerdem fördern die Biguanide die Aufnahme von Zucker in die Muskulatur und gleichzeitig bremsen sie den Appetit;

c) Glitazone verbessern die Empfindlichkeit der Zellen für Insulin, wenn dieses noch produziert wird - hierdurch wird der Blutzuckerspiegel wirksam gesenkt;

d) Glinide regulieren den Blutzucker nach einer Mahlzeit dadurch, dass sie über einen speziellen Mechanismus die kurzfristige Insulinfreisetzung aus den Beta-Zellen der Bauchspeicheldrüse anregen,

e) Sulfonylharnstoffe, die die Blutzucker-Schwelle absenken, ab der die Beta-Zellen der Bauchspeicheldrüse Insulin freisetzen.

Neben den oralen Antidiabetika kann es notwendig werden, dass dem Patient Insulin zugeführt wird. Hierbei werden individualisierte Therapieformen durchgeführt, die auf die Bedürfnisse der Patienten zugeschnitten sind.

US2003/0004095 beschreibt die Verwendung von Molkeprotein-Hydrolysat zur Behandlung von metabolischem Syndrom oder von Diabetes.

Die Behandlung des Metabolischen Syndroms beruht im wesentlichen darauf, dass die Patienten durch eine Ernährungsumstellung und vermehrte Bewegung eine Gewichtsabnahme erreichen. Oft sind jedoch zusätzlich Medikamente zur Blutdruck-, Blutzucker- und Blutfettsenkung notwendig. Schätzungen hinsichtlich der Kosten, die jährlich in den USA durch direkte Aufwendungen und indirekte Kosten durch den Produktivitätsverlust durch die oben genannten Behandlungsformen anfallen, belaufen sich auf insgesamt 98 Milliarden Dollar - bei steigender Tendenz.

Deshalb ist es notwendig, Mittel und Wege zur Prävention und Behandlung des Metabolischen Syndroms oder des Typ 2-Diabetes zur Verfügung zu stellen. Des weiteren werden Mittel zur Verhinderung oder Verzögerung der Verschlechterung der Symptome sowie zu deren Linderung oder Heilung benötigt. Insbesondere wird eine verbesserte Behandlung des Metabolischen Syndroms benötigt, um so einer weiteren Progression in den Typ 2-Diabetes entgegen zu wirken.

Unter dem Begriff "Prävention" wird folgend verstanden, dass das Entstehen eines Symptoms oder einer Erkrankung verhindert wird. Unter dem Begriff "Behandlung" wird nachfolgend jede Form einer Behandlung zur Verbesserung der Krankheitssymptome, der Verzögerung der Progression der Krankheit, der Regression der Krankheit oder der Linderung der Krankheitssymptome verstanden.

**Beschreibung der Figuren**

Die folgenden Figuren zeigen die Ergebnisse von Tierversuchen mit Süßmolkepermeat zur Ermittlung verschiedener Parameter, die für Symptome des Metabolischen Syndroms oder des Diabetes relevant sind.

Figur 1 zeigt die Futteraufnahme und Trinkmenge im Beobachtungszeitraum von 6 Wochen bei Süßmolkepermeat (SMP)-behandelten Tieren und deren Kontrollen.

Figur 2 zeigt die Entwicklung der Körpermasse und den Blutglucoseverlauf im Beobachtungszeitraum bei Süßmolkepermeat (SMP)-behandelten Tieren und deren Kontrollen.

Figur 3(a) stellt die Konzentration von nicht veresterten Fettsäuren (NEFA; non-esterified-fatty-acids) und von Insulin im Blut der Versuchstiere vor Behandlungsbeginn, nach 3 Wochen und nach 6 Wochen Behandlung mit oder ohne SMP dar.

Figur 3(b) stellt die Konzentration von Gesamt- und HDL-Cholesterol im Blut der Versuchstiere vor Behandlungsbeginn, nach 3 Wochen und nach 6 Wochen Behandlung mit oder ohne SMP dar.

Figur 3(c) stellt die Konzentration von LDL-Cholesterol und Triglyzeriden im Blut der Versuchstiere vor Behandlungsbeginn, nach 3 Wochen und nach 6 Wochen Behandlung mit oder ohne SMP dar.

Figur 3(d) stellt die Konzentration an C-reaktivem Protein im Blut der Versuchstiere vor Behandlungsbeginn, nach 3 Wochen und nach 6 Wochen Behandlung mit oder ohne SMP dar.

Figur 3(e) stellt die Leukozytenzahl der Versuchstiere im Behandlungszeitraum von 6 Wochen bei Behandlung mit oder ohne SMP dar.

Figur 4(a) stellt den Blutglukoseverlauf im oralen GlucoseToleranz-Test (oGTT) und die Glukoseüberschreitungsflächen vor Behandlungsbeginn dar.

Figur 4(b) stellt den Verlauf der Serum-Insulinkonzentration im oGTT und die Insulin-Überschreitungsflächen vor Behandlungsbeginn dar.

Figur 5(a) stellt den Blutglukoseverlauf im oGTT und die Glukoseüberschreitungsflächen nach 3 Wochen Behandlung dar.

Figur 5(b) stellt den Verlauf der Serum-Insulinkonzentration im oGTT und die Glukoseüberschreitungsflächen nach 3 Wochen Behandlung dar.

Figur 6(a) stellt den Blutglukoseverlauf im oGTT und die Glukoseüberschreitungsflächen nach 6 Wochen Behandlung dar.

Figur 6(b) stellt den Verlauf der Serum-Insulinkonzentration im oGTT und die Glukoseüberschreitungsflächen nach 6 Wochen Behandlung dar.

Figur 7 stellt das Blutglukose- und Laktat-Tagesprofil vor Behandlungsbeginn dar.

Figur 8 stellt das Blutglukose- und Laktat-Tagesprofil nach 6 Wochen Behandlung dar.

**Detaillierte Beschreibung der Erfindung**

Es wurde überraschend gefunden, dass die Verabreichung von Molkenpermeat an Tiere, die als Modell für das Metabolische Syndrom und den Typ 2-Diabetes anerkannt sind, zur Verhinderung der Glucoseintoleranz und der Verhinderung der Insulinresistenz sowie zu einer Verminderung der TriglyceridKonzentration im Serum führt.

Erfindungsgemäß wird somit gemäß Anspruch 1 die Verwendung einer pharmazeutischen Zusammensetzung zur Verfügung gestellt, die Molkenpermeat umfasst. Diese pharmazeutische Zusammensetzung wird zur Prophylaxe, Behandlung oder Verhinderung der Hypertriglyceridämie, der Glucoseintoleranz und der Insulinresistenz beim Metabolischen Syndrom oder dem Typ 2-Diabetes sowie bei daraus entstehenden Folgeerkrankungen bei Säugern, bevorzugt beim Menschen, verwendet.

Beispiele für Folgeerkrankungen, die mit dem Metabolischem Syndrom oder dem Diabetes einhergehen, sind Gefäßerkrankungen, Koronarinsuffizienz, arterielle Verschlusskrankheiten, Mykokardinfarkt, Xanthomen, Bauchbeschwerden, Milz-Leber-Vergrößerung, Pankreatitis, Netzhaut-Lipämie, und/oder Schlaganfall.

Molke fällt bei der Käseherstellung aus Milch an. Erfindungsgemäß kann Kuhmilch, Ziegenmilch, Schafsmilch, Büffelmilch und Kamelmilch zur Molkegewinnung verwendet werden, nachdem das in der Milch vorhandene Casein ausgefällt wurde. Nach Art der Gewinnung unterscheidet man Süßmolke, die als Milchserum nach enzymatischer Ausfällung von Casein durch Labferment entsteht, und Sauermolke, die nach Abtrennung des Caseins durch Säurefällung gewonnen wird. Die pH-Wert-Grenze zwischen Süß- und Sauermolke ist nicht ganz scharf umrissen und liegt allgemein über bzw. unter einem Bereich von 5,6 bis 5,9. Bevorzugt wird im Zusammenhang dieser Erfindung Süßmolke verwendet.

Die erfindungsgemäß verwendete Molke enthält alle wasserlöslichen Komponenten der Milch, sofern diese nicht durch Lab oder Säure ausgefällt werden. In einer besonderen Ausführungsform wird Süßmolke verwendet, die ca. 4,9% Lactose, 0,8% Protein, 0,5% Mineralstoffe, 0,2% Milchfett und 0,2% Milchsäure enthält.

Aus Molke kann mittels Ultrafiltration (Membranverfahren, mittlere Porengröße: 25 bis 100 kDalton) das Molkeneiweiß abgetrennt werden. Diese "entweißte" Molke besteht zu etwa 95% aus Wasser und kann durch Sprühtrocknen zu einem Pulver weiterverarbeitet werden kann. Dieses Pulver wird hier als Molkenpermeat bezeichnet. In der vorliegenden Erfindung wird Molkenpermeat verwendet, das aus Süßmolke gewonnen wurde. Die darin durchschnittlich enthaltenen Bestandteile sind 84.9% Lactose, 4.5% Protein, 0.1% Fett und 7.5% Mineralstoffe. Beim Rest handelt es sich um nichtabgetrenntes Wasser. Bei diesen Mengenangaben handelt es sich um Durchschnittswerte, die je nach Herstellungsweise um 5-10% (relativ) variieren können. Molkenpermeat kann als solches, oder nach (Teil-)Hydrolyse der Lactose verwendet werden. Bevorzugte Darreichungsformen der Molke sind Sirups oder Pulver, aber auch andere Darreichungsformen sind möglich.

Erfindungsgemäß wird die Süßmolke teilhydrolysiert verwendet.

In einer weiteren erfindungsgemäßen Ausführungsform hat sich eine Mikroverkapselung der pharmazeutischen Zusammensetzung, die Molkenpermeat umfasst, als besonders vorteilhaft erwiesen. Die Mikroverkapselung kann wie unter anderem in den Patent-Offenlegungsschriften DE 198 54 749 A1 und DE 100 08 880 A1 und dem Gebrauchsmuster DE 296 23 285 U1 beschrieben, erfolgen. Dabei wird die Verbindung zum Beispiel in einer Hülle aus einem Polysaccharid, wie z.B. Alginat, fest eingeschlossen. Damit der möglicherweise unverdauliche Hüllstoff eine Freisetzung der Verbindung nicht verhindert und dadurch eine ernährungsphysiologische Nutzung durch den Organismus unmöglich macht, kann eine verdauliche Komponente, wie z.B. Stärke der Umhüllung beigefügt werden. Durch geschickte Wahl und/oder Kombination der lösliche und unlöslichen Umhüllungskomponenten kann so die Abgabe der mikroverkapselten pharmazeutischen Zusammensetzung in verschiedenen Bereichen des Verdauungstrakts gezielt gesteuert werden. Eine abgestufte Freisetzung im Darm, z.B. eine Freisetzung von 50 bis B0 Gew.-%, bevorzugt von 60 bis 70 Gew.-%, insbesondere von 62,5 Gew.-% im Dünndarm, und eine Freisetzung von 20 bis 50 Gew.-%, bevorzugt von 30 bis 40 Gew.-%, insbesondere von 62,5 Gew.-% im Dickdarm ist ein mögliche Art der gezielten Freisetzung. Ein weiterer vorteilhafter Effekt kann durch eine verlängerte Haltbarkeit durch Schutz der verkapselten Verbindung z.B. vor Umwelteinflüssen erzielt werden.

Des weiteren wird Süßmolkepermeat verwendet, aus dem die Lactose teilweise oder ganz entfernt wurde. Dieses Süßmolkepermeat wird im Zusammenhang mit dieser Erfindung als Lactose-reduziertes Süßmolkepermeat bezeichnet. Lactose-reduziertes. Sußmolkepermeat enthält normalerweise ca. 85 % an Lactose, die jedoch daraus zur weiteren Verwendung gewonnen werden kann. Übrig bleibt daher Lactose-reduziertes Süßmolkepermeat, dass zwischen 0.1 und 80 %, bevorzugt zwischen 1 bis 50 %, noch bevorzugter zwischen 5 und 25 % an Lactose enthält. Besonders bevorzugt ist Lactose-reduziertes Süßmolkepermeat, dass noch 10 bis 20 % an Lactose enthält.

Die vorliegende Erfindung stellt ferner ein Präparat zur Verfügung welches die folgenden Wirkungen hat, wie auch in den folgenden Beispielen gezeigt wird:

- Senkung des Seruminsulinspiegels,
- Verhinderung des Entstehens einer Glucoseintoleranz,
- Verhinderung einer Insulinresistenz,
- Senkung des Inselvolumens der Bauchspeicheldrüse,
- Verringerung des Volumens der β-Zellen im Pankreas,
- Verhinderung der Neogenese von β-Zellen im Pankreasgangepithel,
- Verhinderung der Entwicklung von Hyperinsulinismus,
- Verhinderung einer Pankreasentzündung (Pankreatitis) oder einer Insel- und/oder β-Zell-selektiven Entzündung (Insulitis).

Ein weiteres Ziel ist die Verwendung von Molkenpermeat, zur Prävention und/oder Behandlung des Typ 2-Diabetes, vor allem zur Verzögerung der Progression der Erkrankung, bevorzugt zur Verbesserung von deren Symptomatik.

Es wurde erfindungsgemäß überraschend gefunden, dass es für den Hyperinsulinismus ein morphologisches Korrelat gibt. Das bedeutet, dass es zu einer Vermehrung des β-Zellvolumens, dem Auftreten kleiner β-Zellhaufen im exokrinen Pankreas, dem Nachweis von β-Zellen im Pankreasgangepithel (Hinweis auf eine Neogenese) und eine Erhöhung der Mitoserate der □-Zellen kommt. Dieses morphologische Korrelat spricht für erhöhte Anforderungen an dieses Organ, die partiell durch eine Zellvermehrung kompensiert werden, um beispielsweise die übermäßige metabolisierbare Energie zu verwerten.

Es wurde erfindungsgemäß außerdem unerwartet gefunden, dass die Verabreichung von Molkenpermeat zu einer Verhinderung des Anstiegs des β-Zellvolumens führt, und dass diese Substanzen zu einer Verringerung des Inselvolumens im Pankreas führen. Die Verabreichung der pharmazeutischen Zusammensetzungen und Präparate dieser Erfindung für diese Zwecke ist daher bevorzugt

Es wurde erfindungsgemäß bevorzugt, dass die Verwendung von Molkenpermeat zu einer Senkung der Blutfettwerte führt, insbesondere zu einer Senkung der Triglyceridkonzentration.

Des weiteren wurde überraschenderweise herausgefunden, dass eine Behandlung mit Molkenpermeat bei alleiniger oder kombinierter Anwendung mit weiteren wirksamen Substanzen zu einer teilweise signifikanten Senkung der Seruminsulinspiegel führte. Diese wirksamen Substanzen sind Alpha-GlukosidaseHemmer, Biguanide, Glitazone, Sulfonylharnstoffe, Glinide oder Insulin. Die Verwendung von Molkenpermeat sowie die Verwendung von Präparaten, die neben dem Molkenpermeat noch weitere Wirkstoffe enthalten, wird ebenfalls im Rahmen dieser Erfindung zur Verfügung gestellt, solange die weiteren wirksamen Substanzen keinen negativen Einfluss auf die Wirkung des Molkenpermeats ausüben.

In einer weiteren bevorzugten Ausführungsform wird das Molkenpermeat zur Prävention oder Behandlung des Metabolischen Syndroms odes des Typ 2-Diabetes aus der Milch von Rindern, Ziegen, Schafen, Büffeln, Kamelen oder anderen Milch gebenden Tieren gewonnen.

Eine besonders bevorzugte Ausführungsform ist darauf ausgerichtet, dass die erfindungsgemäßen Zusammensetzungen oder Präparate, die Molkenpermeat enthalten, in Form von Nahrungsergänzungsmitteln vorliegen. Auch dabei ist die Verwendung von (teil-)hydrolysiertem Süßmolkepermeat bevorzugt.

In einer weiteren bevorzugten Ausführungsform wird Molkenpermeat zur Herstellung von diätetischen und/oder angereicherten Nahrungsmitteln verwendet, die geeignete pharmazeutisch annehmbare Zusatzstoffe umfassen. Beispiele für Zusatzstoffe sind dem Fachmann bekannte Farbstoffe, Geschmacksverstärker, Konservierungsmittel, Bindemittel, oder Füllstoffe.

Erfindungsgemäß können die oben genannten Substanzen auch zur Herstellung von Nahrungsmitteln, zum Beispiel diätetischen Nahrungsmitteln und/oder Nahrungsergänzungsmitteln verwendet werden, wodurch diese die Eigenschaft erhalten, das Metabolische Syndrom oder die Progression des Typ 2-Diabetes zu verhindern und/oder die Symptome dieser Indikationen zu lindern oder zu heilen. Beispiele für solche Nahrungsmittel sind Milchprodukte oder Fruchtsäfte, die mit erfindungsgemäßen Zusammensetzungen angereichert sind, aber auch andere Nahrungsmittel sind denkbar.

Bei der Behandlung und Prävention von Symptomen des Metabolischen Syndroms und des Typ 2-Diabetes mit Hilfe Molkenpermeat enthaltender Zusammensetzung oder Präparate wird die Dosierung je nach Krankheitsindikation, Alter, Gewicht und Geschlecht der zu behandelnden Person gegebenenfalls individuell variiert.

Die Konzentration der erfindungsgemäßen Molkenpermeat enthaltenden Zusammensetzungen oder Präparate in diätetischen und/oder angereicherten Nahrungsmitteln kann in Abhängigkeit vom ausgewählten geeigneten Zusatzstoff und/oder Trägerstoff variieren, wobei insbesondere Rücksicht auf die organoleptischen Eigenschaften des Endprodukts genommen wird.

Des weiteren können die erfindungsgemäßen Zusammensetzungen in einem Kombinationspräparat zur Prävention oder Behandlung von Symptomen des Metabolischen Syndroms und des Typ 2-Diabetes enthalten sein. Besonders bevorzugt enthält ein Kombinationspräparat neben den erfindungsgemäßen Zusammensetzungen eine oder mehrere der folgenden Substanzen:

Alpha-Glukosidasehemmer, Biguanide, Glitazone, Glinide, Sulfonylharnstoffe. Die Verabreichung der Kombinationspräparate kann ebenfalls individuell an die Bedürfnisse des Patienten angepasst werden. Erfindungsgemäß können diese Kombinationspräparate einzeln in 2 oder mehr Einzeldosierungen oder zusammen verabreicht werden.

Besonders bevorzugt ist außerdem die Verwendung einer erfindungsgemäßen Zusammensetzung zur Herstellung eines Medikaments, bevorzugt zur Linderung oder Verhinderung der Symptome des Metabolischen Syndroms und der Behandlung und Verhinderung des Entstehens von Typ 2-Diabetes, der Verhinderung von deren Progression sowie von Folgeerkrankungen, die durch das Metabolische Syndrom oder den Diabetes entstehen können.

Die Verwendung der erfindungsgemäßen Kombinationspräparate oder Medikamente zur Linderung/Prävention von Symptomen des Metabolischen Syndroms und des Typ 2-Diabetes sind insbesondere auf die Anwendung am Menschen ausgerichtet, aber die Behandlung anderer Säugetiere wie Hunde, Katzen, Pferde, Schafe, Kühe oder Schweine etc. ist ebenfalls in den Schutzbereich eingeschlossen.

Die erfindungsgemäßen Zusammensetzungen und Präparate werden besonders bevorzugt zur Bekämpfung/Prävention des Wachstums von β-Zellen im Pankreas, der Vermehrung von β-Zellen im Pankreas, dem Anstieg des β-Zell- bzw. Inselvolumens oder der Pankreatitis bei einem Säugetier, insbesondere dem Menschen, verwendet.

Des weiteren ist es besonders bevorzugt, dass die Verwendung der erfindungsgemäßen Zusammensetzungen oder Präparate zu einer Senkung des Seruminsulinspiegels führt.

Ferner wird bevorzugt, dass durch die Gabe der erfindungsgemäßen Zusammensetzungen oder Präparate das Entstehen einer Glucoseintoleranz verhindert wird, oder dass diese gelindert wird.

Bevorzugt wird außerdem, dass die Verabreichung der erfindungsgemäßen Zusammensetzungen oder Präparate zu einem verzögerten Anstieg der morgendlichen Hyperglykämie führt.

Weiterhin wird besonders bevorzugt, dass das Konsumieren der erfindungsgemäßen Zusammensetzungen oder Präparate zu einer Senkung der Blutfettwerte führt, insbesondere der Senkung der Konzentration an Triglyceriden, jedoch ist eine Beeinflussung anderer Blutfette ebenfalls in den Schutzbereich der vorliegenden Erfindung einbezogen.

Erfindungsgemäß wird den pharmazeutischen Zusammensetzungen oder Präparaten, die Molkenpermeat umfassen, kein Calciumlactat hinzugegeben.

Von einer Hypertriglyceridämie (Hyperlipämie) spricht man bei einem erhöhten (> 160 mg/100ml) Triglyceridgehalt im Blutserum. Dieser Zustand kann eine Rolle bei der Entstehung von Arterienerkrankungen und der Entwicklung einer koronaren Herzkrankheit spielen (Vega und Grundy, Adv. Exp. Med. 243, 311 (1989)). Zusätzlich ist eine schwere Hypertriglyceridämie (>1.000 mg/dl) mit Chylomicronämie verbunden und sie ruft akute Pankreatitis hervor (siehe K. Soergel, Acute Pancreatitis, in Gastronintestinal Disease 91, 3. Ausgabe (Sleisenger, M.H. und Fordtran, J.S., Hrsg.), W.B. Saunders Company, Philadelphia, Pa., 1983, S. 1462-1485; und Brown, M.S. und Goldstein, J.L., Drugs used in the Treatment of Hyperlipoproteinemias, in Goodman and Gillman's, The Pharmacological Basis of Therapeutics 34, 7. Ausgabe, (Macmillan Publishing Co., New York, 1985, S. 827-845). Ernsthafte Erhöhungen der Chylomicrone rufen auf direktem Weg eine Pankreatitis hervor, die durch Triglyceridverringerung verhindert werden könnte (U.S. Department of Health and Human Services, NIH-Publication Nr. 89-2925, S. 74-77, Jänner 1989, "Report of the Expert Panel on Detection, Evaluation and Treatment of High Blood Cholesterol in Adults"). Es hat sich außerdem gezeigt, dass das Risiko eines Schlaganfalls bei Patienten mit koronaren Herzkrankheiten durch Verringerung des Triglyceridgehalts im Plasma vermindert werden kann (D. Tanne, et al.; Circulation 2001, 104, 2892-2897). Weiterhin ist bei Typ 2-Diabetikern die Lebenserwartung gegenüber Nichtdiabetikern um ein Drittel verkürzt. Dies steht vermutlich in direktem Zusammenhang mit einem erhöhten Triglyceridspiegel (Diabety Care; 2001- 24, 1335-1341). Es ist daher wünschenswert im Rahmen der Behandlung von Symptomen des Metabolischen Syndroms und des Typ 2-Diabetes auch ein Verfahren zur Verringerung von Plasmatriglyceriden bei Patienten mit Hypertriglyceridämie bereitzustellen.

Besonders bevorzugt ist die Verabreichung der erfindungsgemäßen Zusammensetzungen oder Präparate auf oralem Wege, aber auch andere Darreichungsmöglichkeiten sind in den erfindungsgemäßen Schutzbereich einbezogen, z.B. parenteral, intravenös, etc.

In einer besonders bevorzugten Ausführungsform werden Bestandteile der erfindungsgemäßen Zusammensetzungen oder Präparate verabreicht, um die Symptome des Metabolischen Syndroms oder des Typ 2-Diabetes zu verhindern, bzw. um diese zu lindern oder zu heilen.

Die Konzentration an Molkenpermeat in einem pharmazeutischen oder Nahrungsergänzungsmittelpräparat liegen zwischen 0,01% und 99,99%, bevorzugt zwischen 0,1 und 99,9%, bevorzugter zwischen 1 und 99%, insbesondere bevorzugt zwischen 1 und 80%, noch bevorzugter zwischen 10 und 80%, am meisten bevorzugt zwischen 10 und 50% bezogen auf das Gesamtgewicht des Nahrungsergänzungsmittels und/oder des pharmazeutischen Präparats.

Die erfindungsgemäßen Nahrungsergänzungsmittel, bzw. die erfindungsgemäßen pharmazeutischen Präparate können ein oder mehrmals täglich eingenommen bzw. verabreicht werden, z.B. morgens, mittags, abends, zusammen, vor oder nach den Mahlzeiten, aber auch andere Darreichungsschemata sind vorstellbar.

Die Erfindung wird im Folgenden durch Experimente näher erläutert, welche nicht den Bereich der Erfindung weiter einschränken sollen.

## Experimenteller Teil

Tiermodelle/Prüfsysteme

Als Tiermodell zur Untersuchung der Wirkung auf die eingeschränkte Glucosetoleranz, den gestörten Fettstoffwechsel, auf Risikomarker kardiovaskulärer Veränderungen und Entzündungsparameter wurden Zucker-Ratten eingesetzt.

Die Zucker-Ratte ist als Spontanmutation im 13M Stamm von Theodore und Lois Zucker im Laboratory of Comparative Pathology in Stow, Massachusetts (Phänotyp fa = fatty), die zum sogenannten "obese"- bzw. "fatty"-Phänotyp führte, entstanden. Beide Begriffe kennzeichnen Ratten, die übergewichtig und fettsüchtig sind.

Die Zucker-Ratte entwickelt schon frühzeitig eine Hyperphagie, woraus ein rapider Anstieg der Körpermasse (Übergewicht) resultiert. Schon im Alter von 5 bis 6 Wochen kommt es zum Entstehen von Hypertriglyzeridämie, Hyperlipämie, Insulinresistenz, was mit der Entwicklung von Glucoseintoleranz und Hyperinsulinämie einhergeht. Im Alter von 12 Wochen wird die Glucosetoleranzstörung manifest und es kann zur Entwicklung einer Hyperglykämie und damit zur Manifestation eines Typ 2-Diabetes ähnlichen Krankheitsbildes Typ 2-Diabetes gehen in diesem Tiermodell mit histopathologischen Veränderungen der Pankreatischen Inseln einher. Darüber hinaus wurden in diesem Tiermodell auch Veränderungen des Herz-Kreislauf-Systems berichtet. So wurde bei 5 bis 7 Monate alten Tieren eine Erhöhung des Blutdrucks gemessen (Yoshioka, Metabolism Vol. 42 (1), 1993, S. 75 bis 80).

Die Untersuchungen des Einflusses von Molkenpermeat zur Bekämpfung des metabolischen Syndroms wurden an sogenannten WOK.W-Ratten durchgeführt. Die WOK.W-Ratte ist ein Tiermodell, das eine polygenetische Störung vererbt und fast alle Symptome des Metabolischen Syndroms entwickelt, wobei die männlichen Tiere diese Symptome stärker exprimieren. Die spezifischen Eigenschaften der WOK.W-Ratten sind zum Beispiel beschrieben in:

- P.Kovacs et al., Ann.N.Y.Acad.Sci., 1997, 827, 94-99;
- P.Kovacs et al., Biochem.Biophsy.Res.Commun., 2000, 660-665;
- J.van den Brandt et al., Int.J.Obesity, 2000, 24, 1618-1622;
- J.van den Brandt et al., Metabolism 49, 2000, 1140-1144.

Dieses Modelltier ist dafür geeignet, eine exogene Modulierung in Form einer Interventionsstudie bei der Untersuchung des Metabolischen Syndroms vorzunehmen, zumal vorläufige Untersuchungen belegen, dass die Erkranhungsinzidenz beispielsweise durch eine fettreiche Ernährung verändert werden kann.

Tierhaltung:

Die Tiere wurden unter Semi-Barriere-Bedingungen unter einem 12:12 h Licht-Dunkel-Rhythmus (Licht von 06:00 bis 18:00 Uhr) gehalten. Der Zugang zu Futter (RM-Standard-Diät, Sniff, Soest, Deutschland) und Tränkwasser waren ihnen ständig möglich.

Tabelle 1:

| Verabreichungsmenge der jeweiligen Substanzen: | | |
|---|---|---|
| **Testsubstanz** | **Dosis** | **Applikation** |
| Süßmolke-Permeat (SMP) | 25 g/l; entsprechend etwa: 1,5 g/kg/Tag | Gelöst in angesäuertem Tränkwasser |
| Kontrolle | | Angesäuertes Tränkwasser |

Substanzapplikation

Die Substanzen wurden in angesäuertem Tränkwasser gelöst (25 g/l) und den Tieren ad libitum an Stelle des angesäuerten Tränkwassers ohne Zusatz angeboten. Die Kontrollgruppe erhielt reines angesäuertes Tränkwasser (pH: 2,65) Leitungswasser wird in der Versuchstierhaltung zur Keimminderung mit Salzsäure angesäuert). Die Trinkmenge wurde täglich bilanziert und das Tränkwasser stets frisch angesetzt.

Analytische Verfahren

- Die Blutglucose- sowie die Laktat-Konzentration wurden mittels Glucoseanalyzer (Super GL Ambulance, Ruhrtal Labortechnik, Deutschland) gemessen.
- Die Bestimmung der Konzentration des immuno-reaktiven Insulins (IRI) erfolgte mittels Ratten-Insulin spezifischem Radio-Immuno-Assay (Linco Research, Inc.; USA) .
- Die Bestimmung von Gesamtcholesterol, HDL-Cholesterol, LDL-Cholesterol und Triglyzeriden erfolgte photometrisch.
- Das C-reaktive Protein wurde turbidimetrisch bestimmt.
- Die Bestimmung der Leukozytenzahl erfolgte mittels Zählkammermethode.

Auswertungs-Verfahren:

• Darstellung der Blutglucose- und Insulin-Verläufe im oralen Glucosetoleranztest oGGT vom Zeitpunkt -10 min vor Glucosegabe bis 120 min danach. Die Mittelwertskurven für die Untersuchungsgruppen wurden für den jeweiligen Behandlungszeitpunkt für jeden Parameter dargestellt. Zur Beurteilung der physiologischen und der Behandlungs-Effekte wurden für die einzelnen Parameter die reaktiven und absoluten Überschreitungsflächen, entsprechend Studienprotokoll für Blutglucose und Insulin berechnet (G-AUC und I-AUC von 0-120 min: Figuren 4(a) und (b), 5(a) und (b), 6 (a) und (b)
Dazu erfolgte die Dokumentation der Daten der Blutglucose- bzw. Insulinkonzentrationen in Abhängigkeit vom Abnahmezeitpunkt. Die reaktive Fläche wurde wie folgt berechnet:

- absolute Fläche: $F_a$= Fläche unter der Kurve der (absolute AUC, area Blutglucose- bzw. Insulinkonzentration under curve) in Abhängigkeit von der Zeit (0-120 min)

- reaktive Fläche: $F_r = F_a - t\ y_o$ (t= Gesamtdauer des (reaktive AUC) Versuches, $y_o$ = basale Konzentration von Blutglucose- bzw. Insulinkonzentration)

Erläuterung zur Berechnung am Beispiel der Blutglucosekonzentration:
Bei einer Gesamtdauer des Versuchs von 120 min und den im Versuchsdesign festgelegten Abnahmezeitpunkten-10, 0, 10, 20, 30, 40, 60, 90, 120 werden die absoluten Flächen von 0 bis 120 min wie folgt berechnet:

$$F_a = (10y_o + 20y_{10} + 20y_{20} + 20y_{30} + 30y_{40} + 50y_{60} + 60y_{90} + 30y_{120})/2$$

$$F_r = F_a - 120y_o$$

Maßeinheiten: G-AUC in mmol x min/l
I-AUC in ng x min/ml

• Paarvergleich: Es wurde die Differenz zwischen Test- und Kontrollsituation gebildet und mittels t-Test geprüft, ob diese von 0 verschieden ist (p<0,05).
• Mittelwertsvergleich mittels t-Test.
• Ausreißertest nach "Verfahrensbibliothek: Versuchsplanung und -auswertung, Band 1, Berlin 1978, Rasch D. u.a., S.

408

Vorgehensweise/Versuchsdesign:

Die Zucker-Ratten wurden im Alter von ca. 14 Wochen eingestallt und nach einer Woche Adaptation wurde mit den Untersuchungen begonnen. Im Alter von 15 Wochen erfolgte zunächst eine Charakterisierung der Tiere hinsichtlich Glucose- und Fettstoffwechselparametern (u.a. Blutglucose, Serum-Insulin-, Cholesterol-, und Triglyzeridkonzentration), Glucosetoleranz (mittels oralem Glucosetoleranztest) sowie Entzündungsmarkern (Leukozytenzahl und C-reaktives Protein). Die dazu erforderlichen Blutproben wurden aus der Schwanzvene entnommen. Danach wurde mit der Behandlung begonnen. Die Bestimmung von Blutglucosekonzentration, Körpermasse, Leukozytenzahl und Futteraufnahme erfolgte kontinuierlich in wöchentlichen Abständen. Die Tränkwasseraufnahme wurde täglich bilanziert. Nach 2 und 6 Wochen Behandlung wurden erneut Glucose- und Fettstoffwechselparameter gemessen sowie die Glucosetoleranz bestimmt.

Zur Bestimmung der Glucosetoleranz wurden nach nächtlichem Futterentzug Glucosetoleranztests durchgeführt. Dabei wurden im Zeitraum von 10 min vor Glucoseapplikation (per Schlündelsonde) bis 120 min nach Glucosegabe (2g/kg) Blutproben zur Bestimmung von Blutglucose (Abnahmezeiten: -10, 0, 10, 20, 30, 40, 60, 90 und 120 min) und Insulinkonzentration (Zeitpunkt der Abnahmen: 0, 20, 40, 60, 90 und 120 min) entnommen. Vor Beginn sowie nach 6 Wochen Behandlung wurde ein Tagesprofil (3h Intervall) der Blutglucose- und Lactatkonzentration erstellt. Nach Abschluss der regulären Beobachtungszeit von 43 Tagen wurde auch nach Durchführung der abschließenden oralen Glucosetoleranztests bis zur Aufarbeitung am 62./63. Behandlungstag das Behandlungsregime beibehalten. Die Tötung der Tiere erfolgte mittels Narkose-Überdosierung. Nach Narkoseeintritt wurden die Pankreata entnommen und in Bouin's-Lösung für die anschließende histologische Untersuchung fixiert und aufbewahrt.

*Probenaufbewahrung:*

Alle Serumproben wurden bis zur Analytik in der Tiefkühltruhe bei -20°C gelagert.

Ergebnisse:

Nachfolgeuntersuchungen

*Futter- und Trinkwasseraufnahme*

• Obwohl die Trinkmenge bei Tieren der mit SMP behandelten Gruppe leicht erhöht waren (Fig. 1), ist die tägliche aufgenommene Menge an Kalorien in allen Gruppen vergleichbar (Fig. 1a). In der täglichen Futteraufnahme unterscheiden sich die Gruppen nicht signifikant (Fig. 1) Die kalkulierte Energieaufnahme am 42. Behandlungstag betrug:

| | |
|---|---|
| SMP-Gruppe: | 87,6 $\pm$ 6,6 kcal/24h |
| Kontrolle: | 87,9 $\pm$ 25,5 kcal/24h |

Unterschiede zwischen den Gruppen ließen sich nicht sichern.

*Körpermasseentwicklung*

- Die Behandlungsgruppen unterscheiden sich in ihrer Körpermasseentwicklung nicht (Fig. 2)

*Verlauf von Blutglucose und Insulin*

- Vor Behandlungsbeginn, d.h. im Alter von 15 Wochen, waren Blutglucosekonzentration (Fig. 2), Insulinkonzentration (Fig. 3a) der Tiere in den Untersuchungsgruppen vergleichbar. Während die morgendliche Blutglucose in der Kontrollgruppe nach Behandlungsbeginn weiter anstieg, wie im Altersverlauf in diesem Tiermodell erwartet, blieb dieser Anstieg in der SMP Gruppe weitgehend aus.

- Die Insulinkonzentration (Fig. 3a) in der mit SMP behandelten Gruppe fielen mit zunehmender Behandlungsdauer im Vergleich zum Zeitpunkt vor Behandlungsbeginn ab (n.s.). Die Insulinkonzentrationen blieben auf unverändertem Niveau (Kontrolle).

*Fettstoffwechselparameter*

- Die Konzentrationen der nicht veresterten Fettsäuren blieben im Beobachtungszeitraum unverändert. Es gab keine Differenzen zwischen den Behandlungsgruppen (Fig. 3a).
- Im Vergleich zur Kontrollgruppe stiegen die Cholesterol-Konzentrationen im Beobachtungszeitraum im Gegensatz zu der SMP-Gruppe an (n.s., Fig. 3b)
- Die Konzentrationen an HDL-Cholesterol blieben im Beobachtungszeitraum unverändert (Fig. 3b). Nur vor Behandlungsbeginn gab es Differenzen zwischen den Gruppen, die jedoch hinsichtlich des Behandlungseffektes keine Bedeutung hatten.
- Der Anstieg der LDL-Cholesterolkonzentration (Fig. 3c), wie er in der Kontrollgruppe zu beobachten war, konnte durch die SMP-Behandlung zwar nicht verhindert werden, er fiel jedoch im Vergleich zur Kontrollgruppe geringer aus (n.s.).
- In der Gruppe der mit SMP behandelten Tiere war eine Tendenz zur Absenkung der Triglyzeridkonzentrationen (Fig. 3c) erkennbar.

*Entzündungspararameter*

- Die Entzündungsparameter C-reaktives Protein (Fig. 3d) und Leukozytenzahl veränderten sich unter den Therapien im Beobachtungszeitraum nicht.

<u>Ergebnisse der oralen Glucosetoleranztests:</u>

• Die Verläufe von Blutglucose und Insulinkonzentrationen während des oralen Glucosetoleranztestes (oGTT) der Gruppen vor Behandlung waren vergleichbar (Fig. 4a und 4b).
• Nach 3 Wochen Behandlung waren keine signifikanten Unterschiede zwischen den Behandlungsgruppen hinsichtlich Verlauf und Überschreitungsflächen von Blutglucose und Insulin nachweisbar (Fig. 5a und b).
• Nach 6 Wochen Behandlung stieg die Blutglucose der SMP Gruppe im GTT deutlich geringer an und nach 120 min Beobachtungszeit fiel sie auf ein signifikant niedrigeres Niveau im Vergleich zur Kontrollgruppe ab. (Fig. 6a und b).
Analyse der Blutglucosekurven (BG, mmol/l) der GTT vor und nach 6 Wochen Therapie, Mittelwert ± SD (*p<0,05 vs. Kontrolle) :

Tabelle 2

| Gruppe | Parameter | Vor Behandlung | Nach 6 Wochen |
|---|---|---|---|
| **SMP** | BG-10 min | 4,94 ± 0,69 | 5,29 ± 0,79 |
| | BG max. | 11,64 ± 0,75 | 12,61 ± 2,10 |
| | BG 120 min | 7,95 ± 1,27 | 8,02 ± 1,42* |
| **Kontrolle** | BG-10 min | 4,84 ± 1,17 | 5,62 ± 0,59 |
| | BG max. | 11,99 ± 2,03 | 14,25 ± 1,58 |
| | BG 120 min | 8,08 ± 2,14 | 10,22 ± 2,19 |

• Die Glucosetoleranz nach 6 Wochen Behandlung, gemessen als Glucoseüberschreitungsfläche (G-AUC) im GTT, war in der mit SMP behandelten Gruppe im Vergleich zur Kontrollgruppe signifikant verbessert. Während im Beobachtungszeitraum die G-AUC in der Kontrollgruppe zunahm, war sie in der SMP behandelten Gruppe vergleichbar mit der vor Behandlungsbeginn.
Auswertung der reaktiven G-AUC 0-120 min (mmol x min/l) vor und nach 6 Wochen Behandlung, Mittelwert ± SD (p<0,05 VS. Kontrolle):

**Tabelle 3**

| Gruppe | Vor Behandlung | Nach 6 Wochen |
|---|---|---|
| **SMP** | 570 ± 75 | 569 ± 145 |
| **Kontrolle** | 597 ± 124 | 780 ± 138 |

**Tabelle 4**:

| Vergleich ausgewählter Parameter von Patienten und WOK.W-Ratten | | |
|---|---|---|
| ***Parameter*** | ***WOK.W-Ratte*** | ***MENSCH*** |
| Fettsucht | +++[1] | +++ |
| Hypertriglyceridämie | +++ | +++ |
| Hypercholesterinämie | + | ++[2] |
| Dyslipoproteinämie | ++ | +++ |
| Vermindertes HDL-Cholesterin | +3 | ++ |
| Hyperleptinämie | +++ | +++ |
| Glucoseintoleranz | ++ | +++ |
| Insulinresistenz | ++ | +++ |
| Hyperinsulinämie | ++ | ++ |
| Hypertonie | + | +++ |
| 1: +++ = sehr stark ausgeprägt, 2:++ = stark ausgeprägt, 3: + = vorhanden | | |

Die obenstehende Tabelle verdeutlicht, warum die WOK.W-Ratte ein höchst geeignetes Tiermodell für das metabolische Syndrom beim Menschen ist.

Pankreas-Morphometrie

Die untersuchten Pankreata wurden im Alter von 16 Wochen, d.h. nach einer Behandlungsperiode von 12 Wochen, entnommen. Die Pankreata wurden in Paraffin eingebettet und mit einer Schnittdicke von 7 μm geschnitten. Jeder zehnte Schnitt wurde entweder mit Hämatoxilin-Eosin gefärbt (zur Darstellung des Inselvolumens und zur Bewertung einer Entzündung oder mit Anti-Insulin-Antikörpern (zur Darstellung des β-Zellvolumens) inkubiert. Zur Bewertung einer Entzündung (Insulitis) wurden 15 Inseln ausgezählt. Die immunhistochemische Reaktion wurde mit APAAP visualisiert (rot gefärbt). Die morphometrische Bewertung der Verteilung der Inselzellen und der Insulin bildenden β-Zellen im Vergleich zu exokrinen Zellen, Bindegewebe und Gefäßen, erfolgte durch Auszählen von 40.000 Punkten eines Netzes, das über das über dem Präparat liegt. Jeweils vier Tiere aus jeder Gruppe wurden bearbeitet.

Tabelle 5:

| Ergebnisse der immunohistochemischen Untersuchung der Pankreata | | | |
|---|---|---|---|
| | **Inselvolumen** | **β-Zellvolumen** | **Non β-Zellvolumen** |
| Kontrolle (n=8) | 1,12 ± 0,06 (1,00-1,53) | 0,98 ± 0,02 (0,86-1,06) | 0,14 ± 0,05 (0,02-0,47) |
| SMP (n=7) | 0,79 ± 0,12 (0,41-1,21) | 0,63 ± ,010 (0,27-0,86) | 0,17 ± 0,04 0,00-0,36 |
| P (zur Kontrolle | < 0,01 | < 0,005 | Nicht signinifikant |

Die Pankreas-Morphometrie der WOK.W-Ratten zeigte, dass für den Hyperinsulinismus ein morphologisches Korrelat gefunden werden konnte (siehe Tabelle 5 oben). Dies äußerte sich in einer Vermehrung des β-Zellvolumens, dem Auftreten kleiner β-Zellhaufen im exokrinen Pankreas, dem Nachweis von β-Zellen in den Epitelien des Pankreasgangs (Hinweis auf eine Neogenese) und einer Erhöhung der β-Zellmitosezahl, die ursächlich an der Entwicklung des Hyperinsulinismus beteiligt sind. Die nachgewiesene Morphologie spricht für eine erhöhte Anforderung an dieses Organ (z.B. durch die Zunahme metabolisierbarer Energie), die partiell durch eine Zellvermehrung kompensiert wurde.

Durch die Applikation von Molkenpermeat im Trinkwasser wird der Anstieg des β-Zellvolumens verhindert bzw. nach einer Behandlungsperiode von 12 Wochen signifikant vermindert

Obwohl Molke hochenergetisch ist, wurde bei keinem der untersuchten Tiere das Auftreten einer Insel- und/ oder β-Zell-selektiven Entzündung (Insulitis) erkennbar.

Untersuchung von Seruminsulinspiegeln in WOK.W-Ratten

Die Untersuchung der Seruminsulinspiegel bei behandelten Ratten zeigten, dass diese über dem Behandlungszeitraum keine Erhöhung oder eine verminderte Erhöhung des Seruminsulinspiegels aufwiesen, wenn sie mit Kontrollratten verglichen wurden.

Tabelle 6: Seruminsulinwerte (ng/ml) behandelter und unbehandelter WOK.W-Ratten im Vergleich zu altersidentischen Kontrollen.

| Gruppe | 8 Wo | 16 Wo | P |
|---|---|---|---|
| Kontrolle | 3,61 ± 0,24 | 6,12 ± 0,61 | <0,01 |
| SMP | 3,77 ± 0,36 | 4,47 ± 0,64 | Nicht signifikant |

Im Beobachtungszeitraum wird die Zunahme n der Glucoseintoleranz durch die Behandlung mit SMP verhindert.

● Die Insulinausschüttung während des oralen Glucosetoleranztestes (gemessen als Insulin-Überschreitungsfläche; I-AUC) war nach 6 Wochen Behandlung in der Gruppe der SMP behandelten Tiere am höchsten.

● Die Insulinausschüttung im oGTT wurde nach 6 Wochen SMP Behandlung am besten stimuliert. Das freigesetzte Insulin führte jedoch nur in der SMP Gruppe zur signifikanten Absenkung der Blutglucose im oGTT.

Tabelle 7:

| Analyse der reaktiven I-AUC 0-120 min (ng x min/l), mean (Mittelwert) ± SD (Standartabweichung): | | |
|---|---|---|
| **Gruppe** | **Vor Behandlung** | **Nach 6 Wochen** |
| **SMP** | 578 ± 667 | 897 ± 120 |
| **Kontrolle** | 711 ± 664 | 745 ± 506 |

Die β-Zellen des Pankreas der Tiere aus der SMP Gruppe waren nach 6 Wochen Behandlung am besten in der Lage, auf den Glucosereiz zu reagieren. Die Tiere waren darüber hinaus weniger Insulin-resistent.

Blutglucose- und Laktat-Tagesprofile:

● Die Blutglucosetagesprofile zeigen zu den Zeitpunkten vor und nach 6 Wochen Behandlung keine signifikanten Unterschiede zwischen den Behandlungsgruppen (Fig. 7 und 8), wobei jedoch die Blutglucosekonzentrationen der 6 Wochen mit SMP behandelte Gruppe im Tagesverlauf am niedrigsten ausfielen.

● Das Laktat-Tagesprofil zeigt zwischen den Gruppen zu einigen Zeitpunkten signifikante Differenzen. Eine Laktazidose konnte jedoch zu jedem Zeitpunkt ausgeschlossen werden (Fig. 7 und 8). Die höheren Laktatkonzentrationen vor Behandlungsbeginn werden als Stressreaktion gedeutet, da ein Gewöhnungseffekt der Tiere an die Blutentnahmen zu diesem Zeitpunkt noch nicht eingetreten sein konnte.

**Patentansprüche**

1. Verwendung von teilhydrolysiertem, Laktose-reduziertem Süßmolkepermeat zur Herstellung einer pharmazeutischen Zusammensetzung zur Prophylaxe oder Behandlung von Symptomen des Metabolischen Syndroms oder des Typ 2-Diabetes bei einem Säuger, wobei die Symptome ausgewählt sind aus Glucoseintoleranz oder Insulinresistenz, und wobei der pharmazeutischen Zusammensetzung kein Calcium-Laktat hinzu gegeben wurde.

**2.** Verwendung gemäß Anspruch 1, wobei die Zusammensetzung mikroverkapselt ist.

**3.** Verwendung gemäß einem der Ansprüche 1 oder 2, wobei die Zusammensetzung in einer oralen Darreichungsform ausgebildet ist.

**4.** Verwendung gemäß einem der Ansprüche 1 bis 3, wobei die orale Darreichungsform einer Lutschtablette, Pulver, Granulat, Sirup oder Saft ist.

**5.** Verwendung gemäß einem der Ansprüche 1 bis 4, wobei die Zusammensetzung ferner ein Zusatzstoff von Lebensmitteln ist.

**6.** Verwendung gemäß Anspruch 5, wobei die Lebensmittel diätetische Lebensmittel oder Nahrungsergänzungsmittel sind.

**7.** Verwendung gemäß einem der Ansprüche 1 bis 6, wobei die Zusammensetzung ferner pharmazeutisch annehmbare Zusatzstoffe und/oder Trägermaterialien umfasst.

**8.** Verwendung gemäß einem der Ansprüche 1 bis 7, wobei der Säuger ein Mensch ist.

## Claims

**1.** Use of partly hydrolysed, lactose-reduced sweet whey permeate for producing a pharmaceutical composition for the prophylaxis or treatment of symptoms of metabolic syndrome or of Type 2 diabetes in a mammal, wherein the symptoms are selected from glucose intolerance or insulin resistance, and wherein no calcium lactate has been added to the pharmaceutical composition.

**2.** Use according to claim 1, wherein the composition is microencapsulated.

**3.** Use according to one of claims 1 or 2, wherein the composition is presented in an oral form of administration.

**4.** Use according to one of claims 1 to 3, wherein the oral form of administration is a sucking tablet, powder, granules, syrup or juice.

**5.** Use according to one of claims 1 to 4, wherein the composition is also an additive of foodstuffs.

**6.** Use according to claim 5, wherein the foodstuffs are dietary foodstuffs or food supplements.

**7.** Use according to one of claims 1 to 6, wherein the composition also comprises pharmaceutically acceptable additives and/or excipients.

**8.** Use according to one of claims 1 to 7, wherein the mammal is a human being.

## Revendications

**1.** Utilisation de perméat de lactosérum doux à teneur en lactose réduite, ayant subi une hydrolyse partielle, pour la préparation d’une composition pharmaceutique pour la prophylaxie ou le traitement de symptômes du syndrome métabolique ou du diabète de type 2 chez un mammifère, dans lequel les symptômes étant sélectionnés d’après le manque de tolérance au glucose ou la résistance à l’insuline, et dans lequel aucun lactate de calcium n’est ajouté à la composition pharmaceutique.

**2.** Utilisation selon la revendication 1, dans laquelle la composition est micro-encapsulée.

**3.** Utilisation selon l’une des revendications 1 ou 2, dans laquelle la composition est réalisée sous une forme d’administration orale.

**4.** Utilisation selon l’une des revendications 1 à 3, dans laquelle la forme d’administration orale est celle d’une pastille à sucer, d'une poudre, d'un granulat, d'un sirop ou d'un jus.

5. Utilisation selon l'une des revendications 1 à 4, dans laquelle la composition est en outre un additif de produits alimentaires.

6. Utilisation selon la revendication 5, dans laquelle les produits alimentaires sont des produits alimentaires diététiques ou des produit de complément d'alimentation.

7. Utilisation selon l'une des revendications 1 à 6, dans laquelle la composition comprend en outre des additifs et/ou des matériaux supports pharmaceutiquement acceptables.

8. Utilisation selon l'une des revendications 1 à 7, dans laquelle le mammifère est un humain.

Fig. 1: Futteraufnahme und Trinkmenge im Beobachtungszeitraum

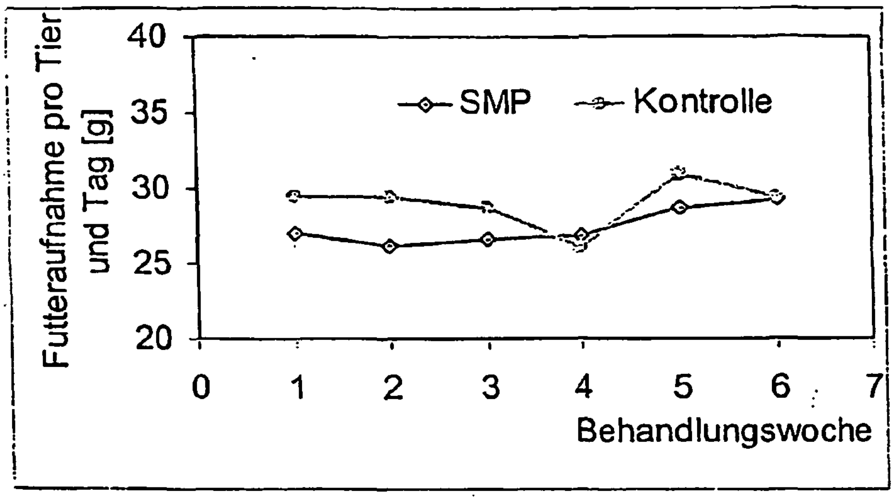


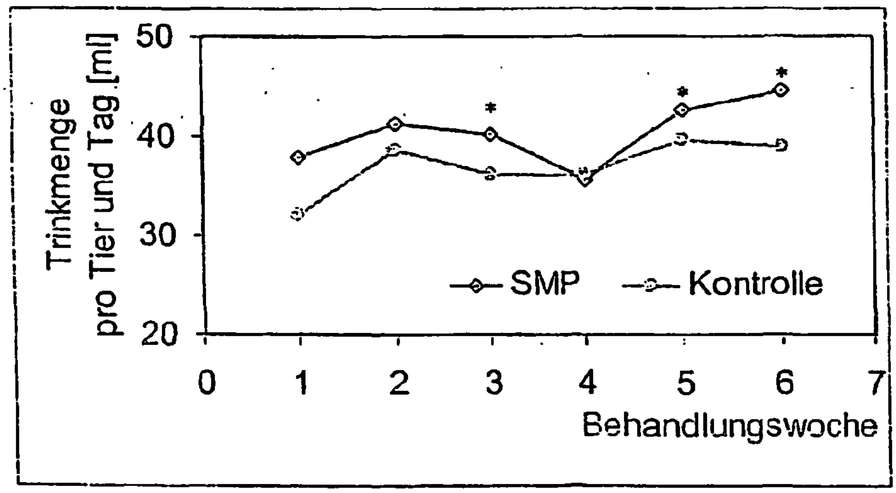


* p<0.05 vs. Kontrolle

**Fig. 2:** Körpermasse und Blutglukosekonzentration im Beobachtungszeitraum (7 Tage vor Behandlungsbeginn bis zu 42 Tagen Behandlung)

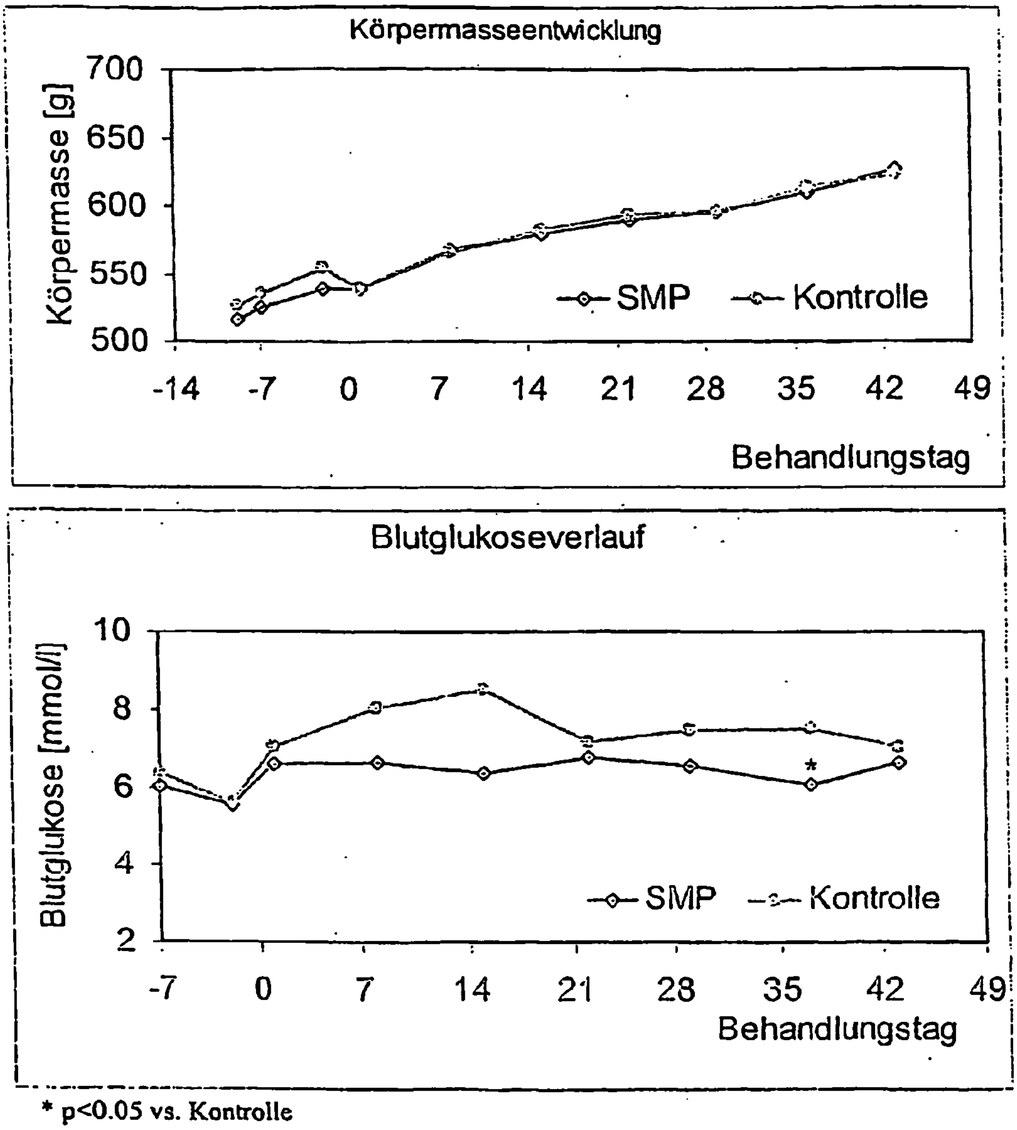


* $p<0.05$ vs. Kontrolle

**Fig. 3a:** Konzentration von NEFA (non-esterified-fatty-acids) und Insulin vor Behandlungsbeginn, nach 3 Wochen und nach 6 Wochen Behandlung mean ± SD

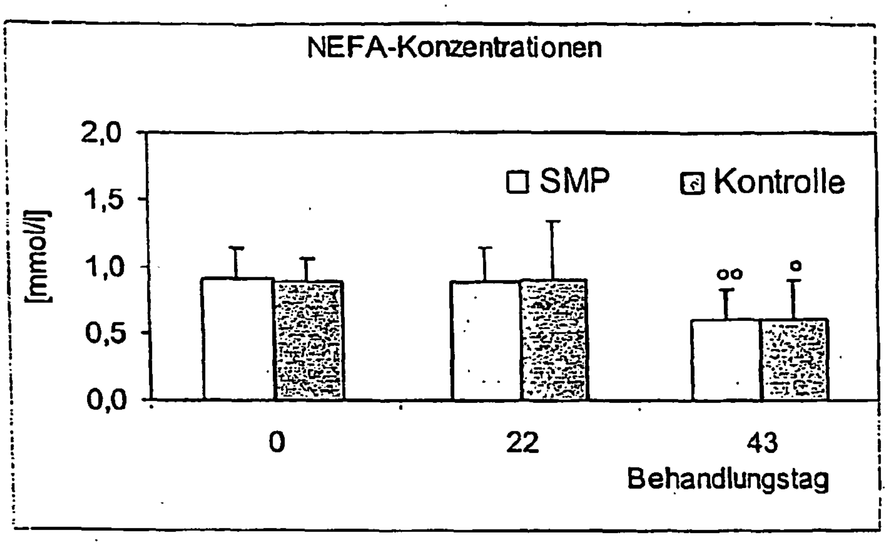


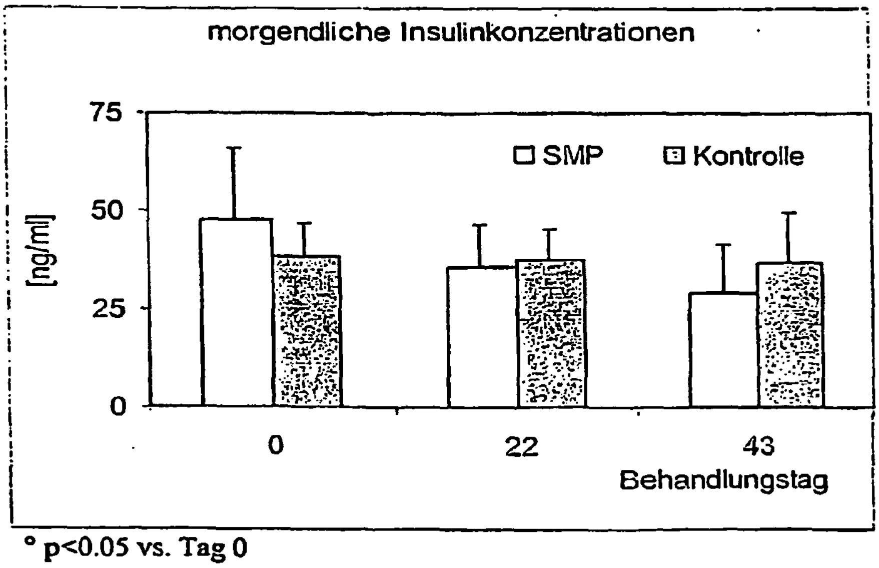


° $p<0.05$ vs. Tag 0
°° $p<0.01$ vs. Tag 0

**Fig. 3b:** Konzentration von Gesamt- und HDL-Cholesterol vor Behandlungsbeginn, nach 3 Wochen und nach 6 Wochen Behandlung, mean ± SD

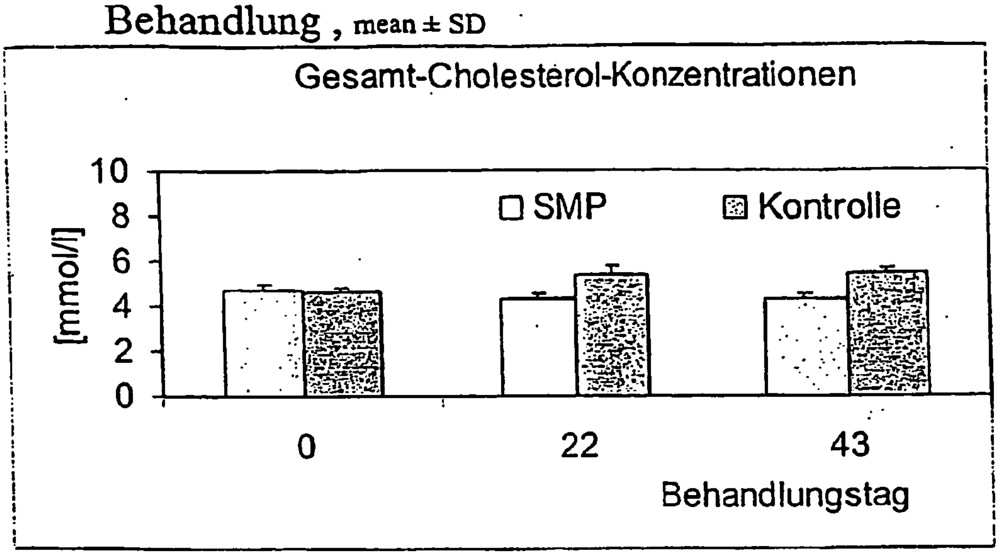


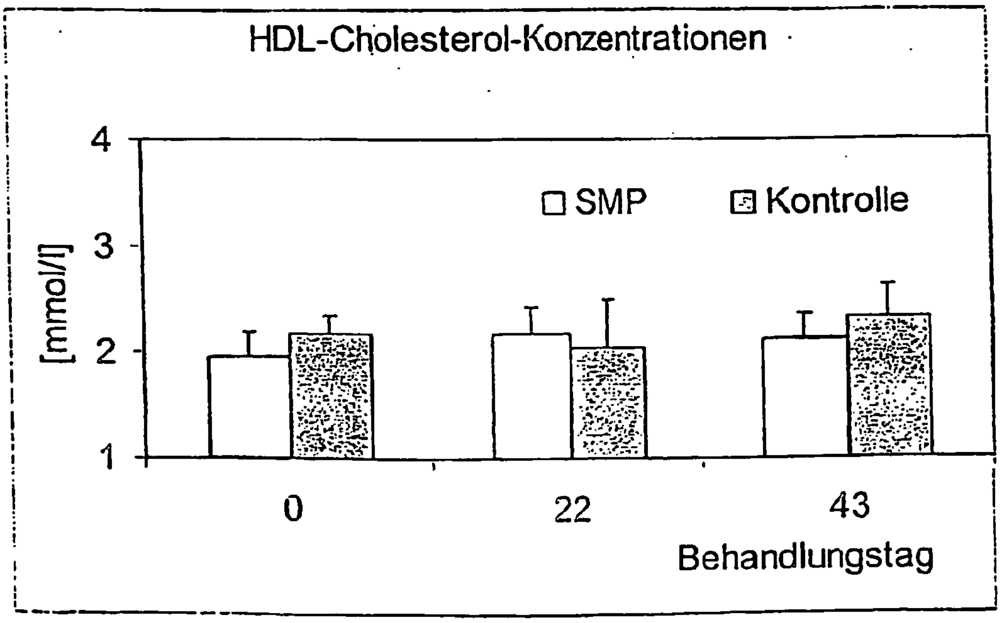

Fig. 3c: Konzentrationen von LDL-Cholesterol und Triglyzeriden vor Behandlungsbeginn, nach 3 Wochen und nach 6 Wochen Behandlung

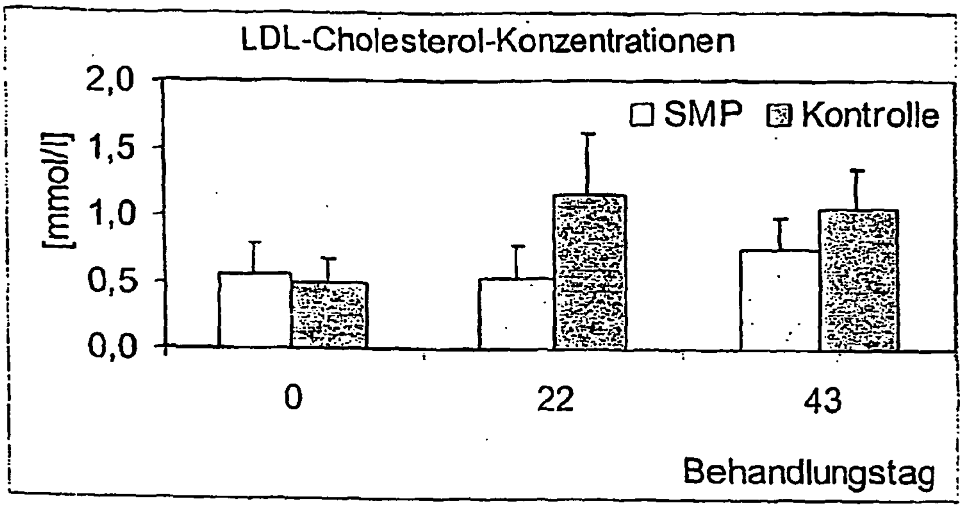


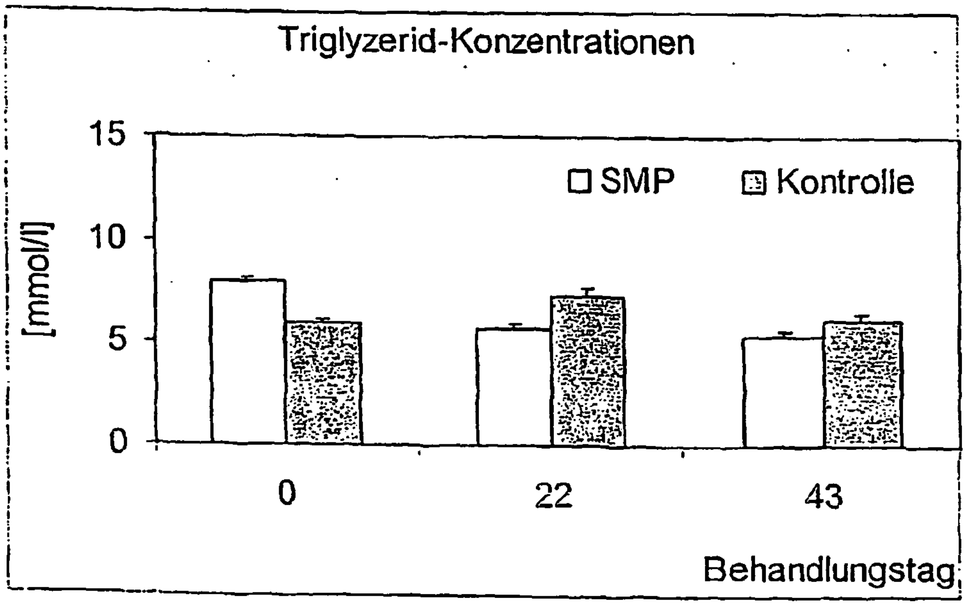

**Fig. 3d:** Konzentrationen von C-reaktivem Protein vor Behandlungsbeginn, nach 3 Wochen und nach 6 Wochen Behandlung , mean ± SD

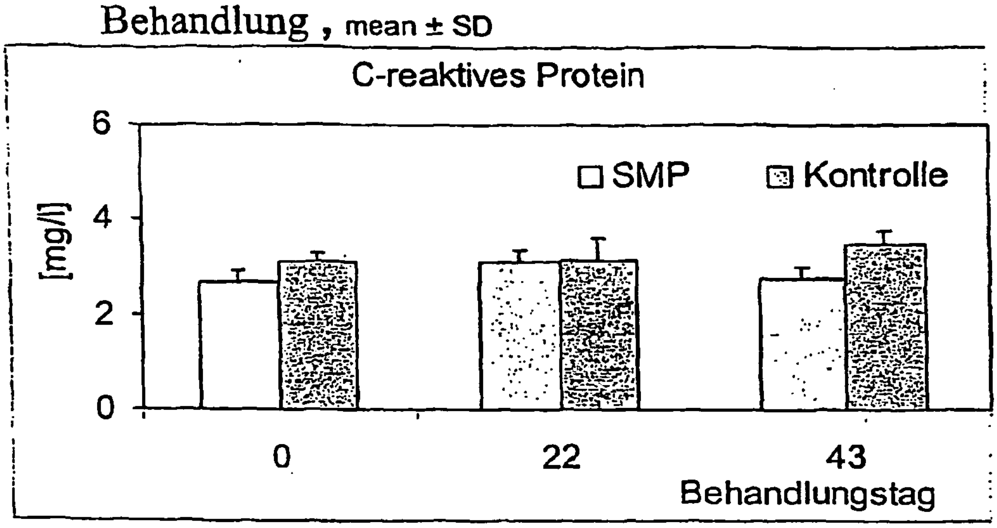

Fig. 3e: Leukozythenzahl im Beobachtungszeitraum von 6 Wochen

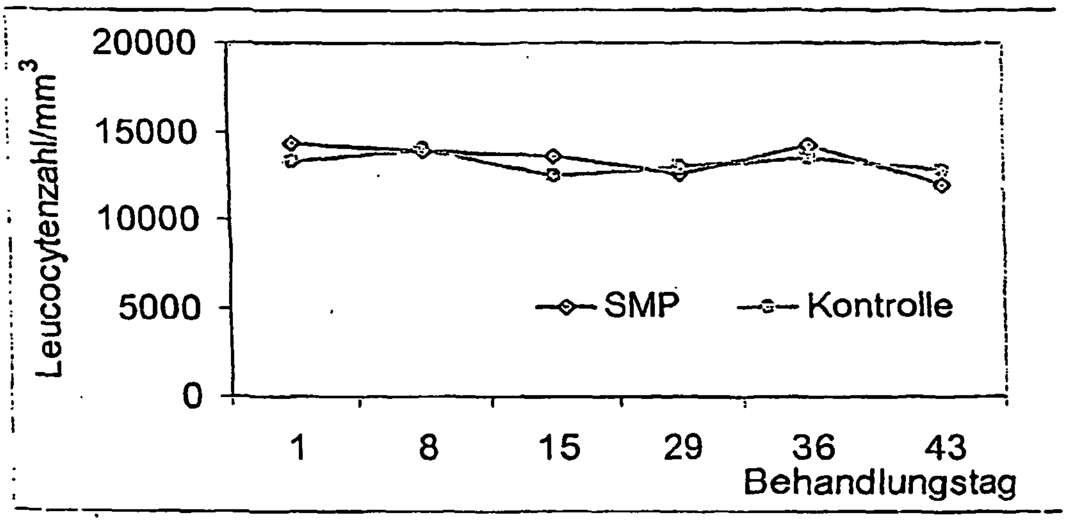

**Fig. 4a:** Blutglukoseverlauf im oGTT und Glukose-Überschreitungsflächen vor Behandlungsbeginn

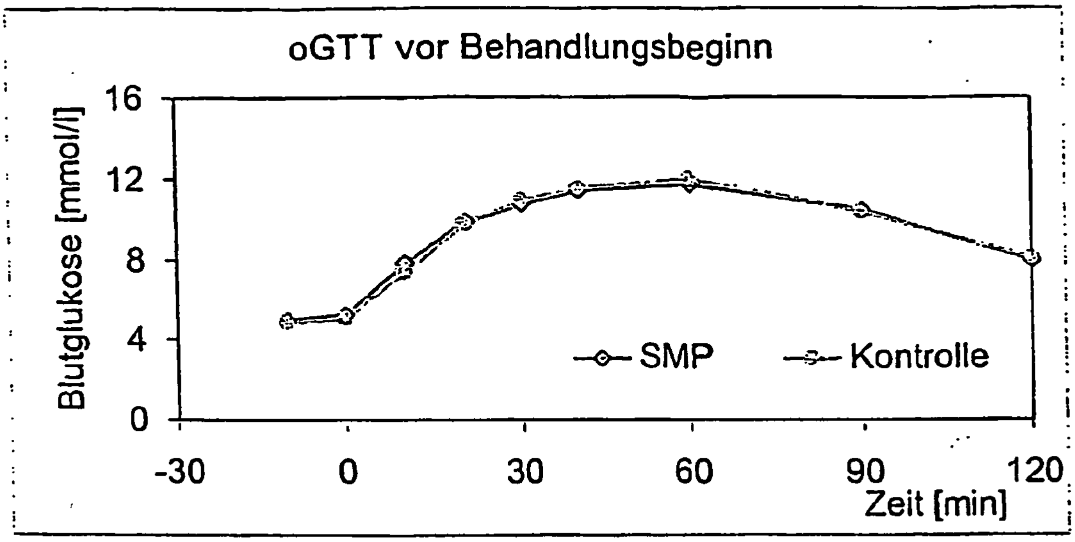


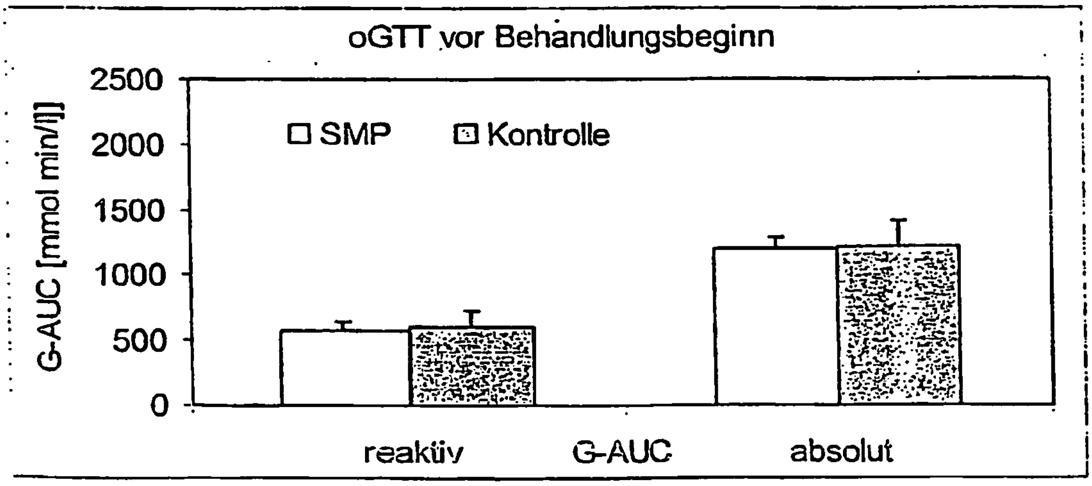


mean ± SD

**Fig. 4b:** Verlauf der Insulinkonzentrationen im oGTT und Insulin- Überschreitungsflächen vor Behandlungsbeginn

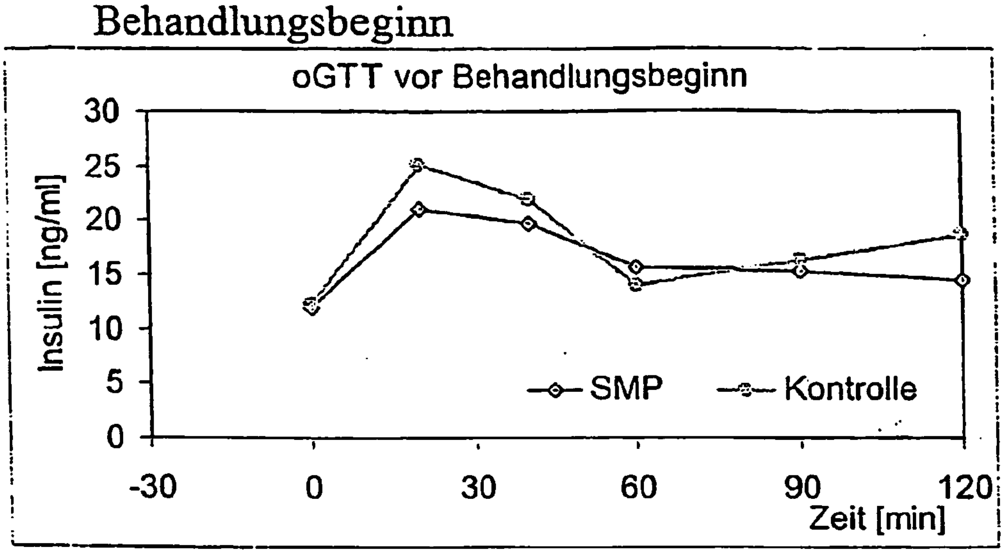


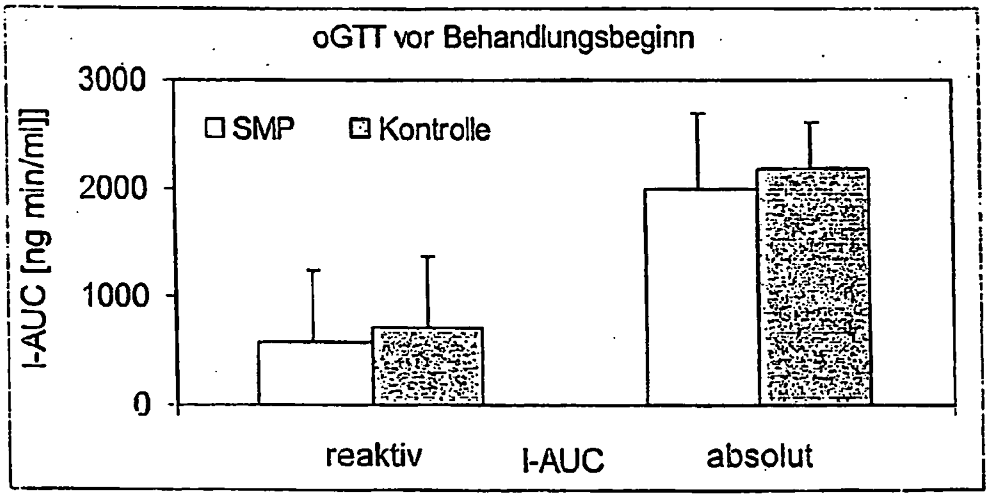


mean ± SD

**Fig. 5a:** Blutglukoseverlauf im oGTT und Glukose-Überschreitungsflächen nach 3 Wochen Behandlung

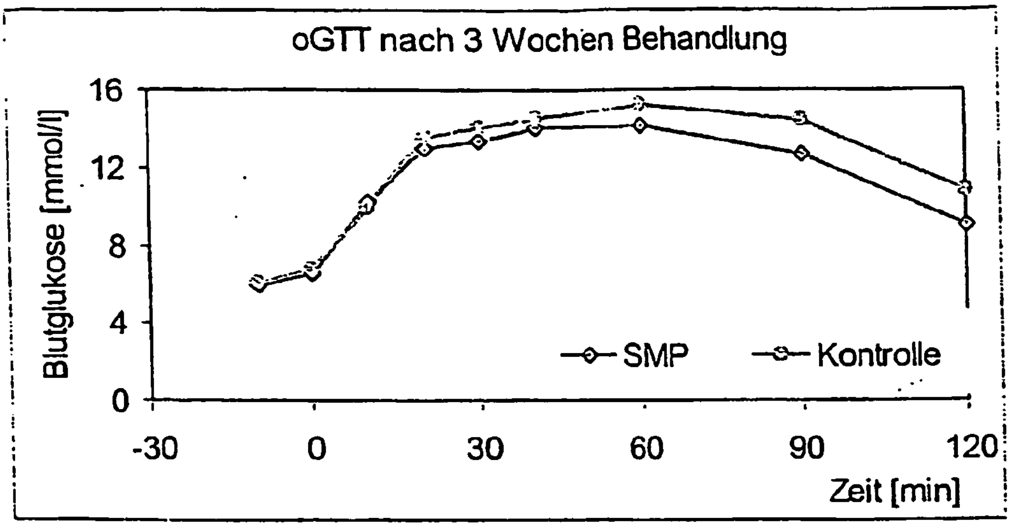


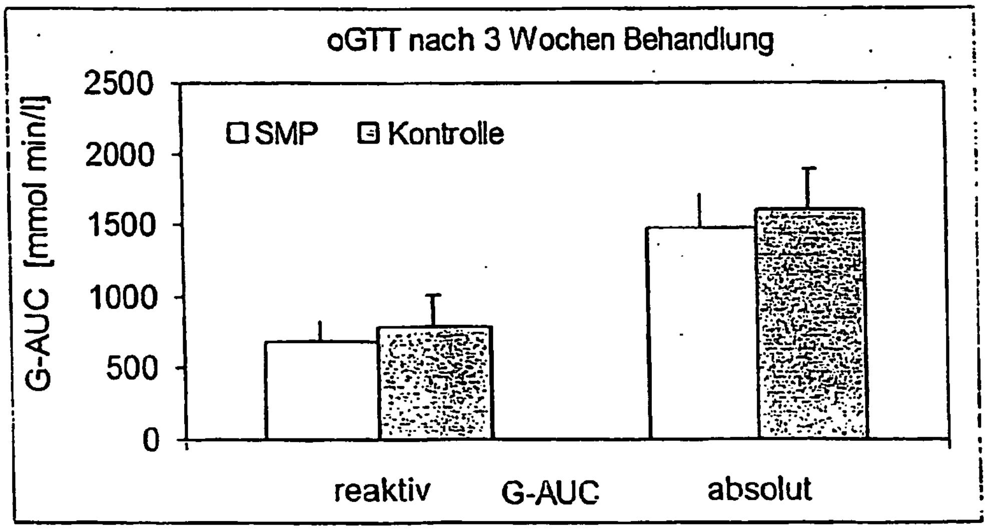


mean ± SD

**Fig. 5b:** Verlauf der Insulinkonzentrationen im oGTT und Insulin-Überschreitungsflächen nach 3 Wochen Behandlung

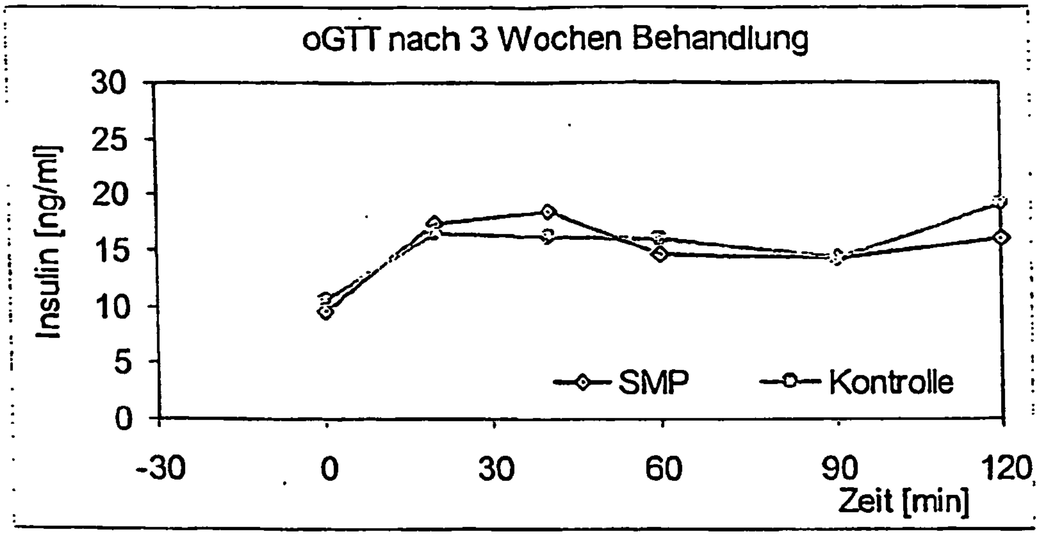


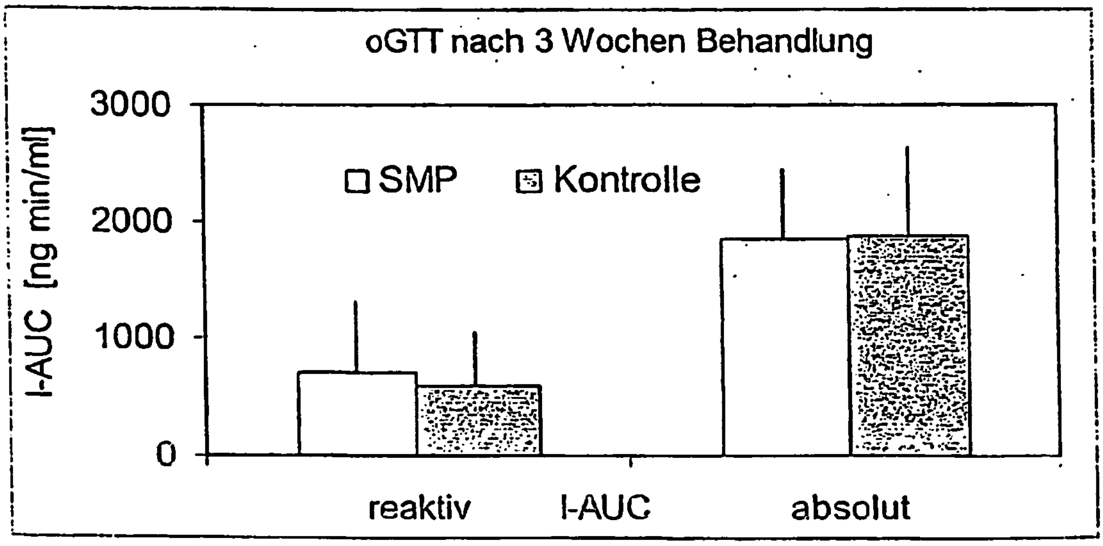


mean ± SD

**Fig. 6a : Blutglukoseverlauf im oGTT und Glukose-Überschreitungsflächen nach 6 Wochen Behandlung**

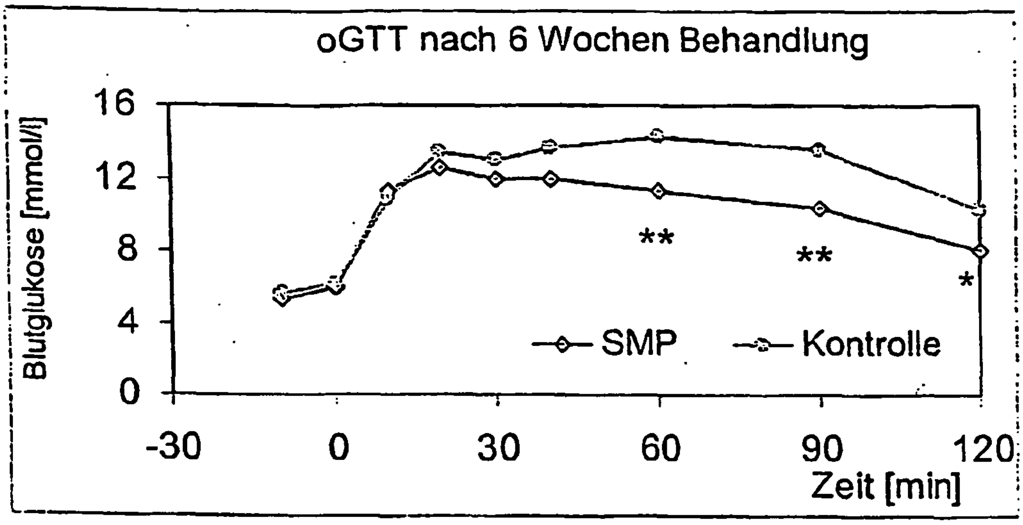


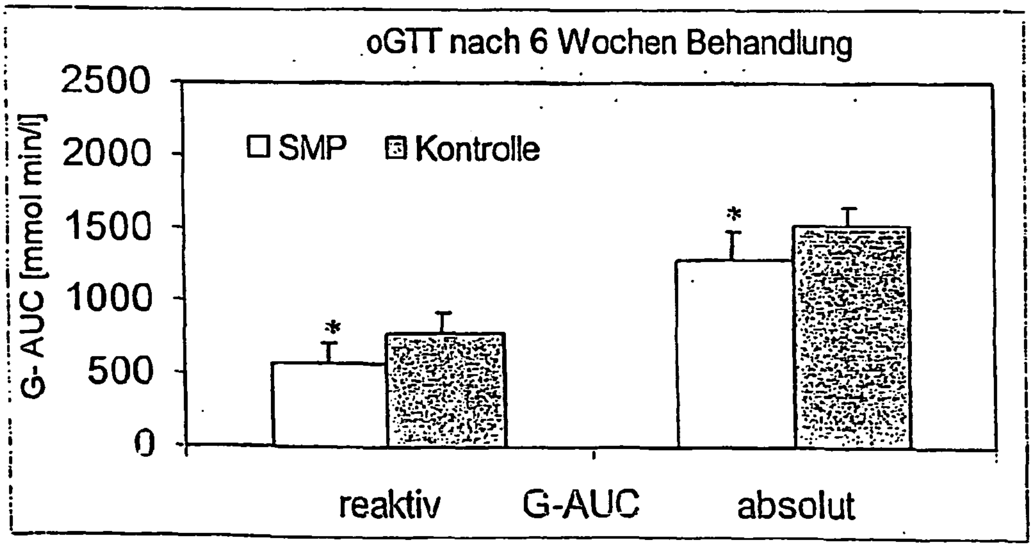


mean ± SD * $p<0.05$ vs. Kontrolle

** $p<0.01$ vs. Kontrolle

**Fig. 6b:** Verlauf der Insulinkonzentrationen im oGTT und Insulin-Überschreitungsflächen nach 6 Wochen Behandlung

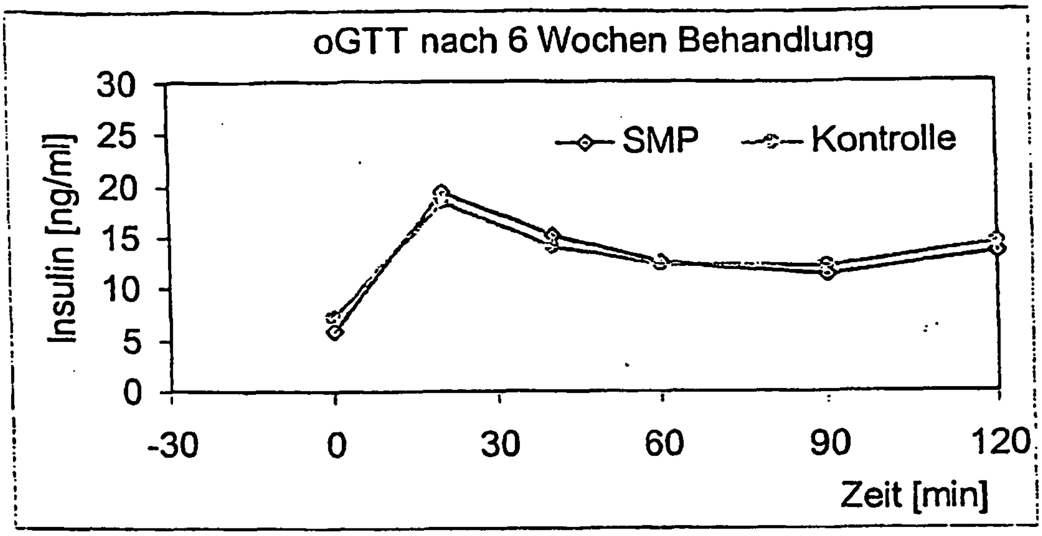


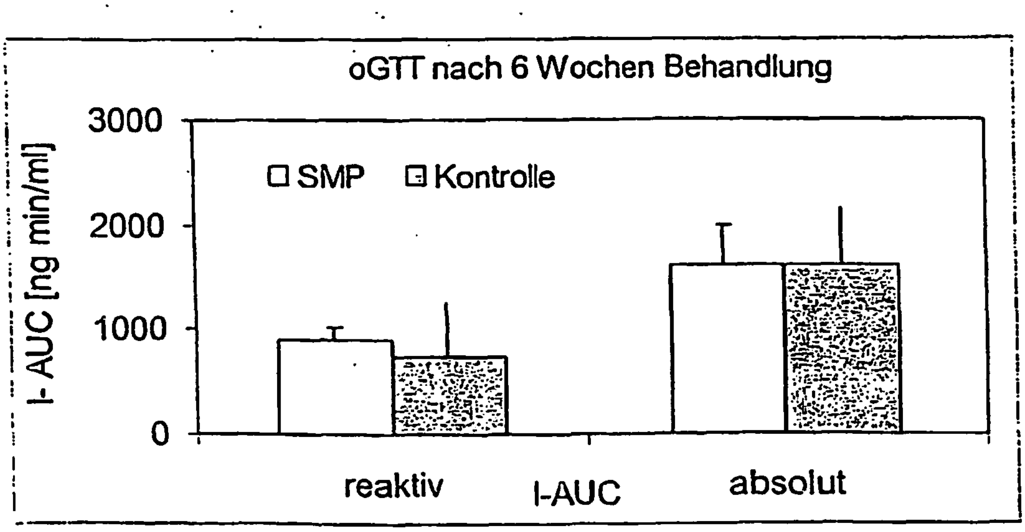


mean ± SD

**Fig. 7 : Blutglukose- und Laktat-Tagesprofil vor Behandlungsbeginn**

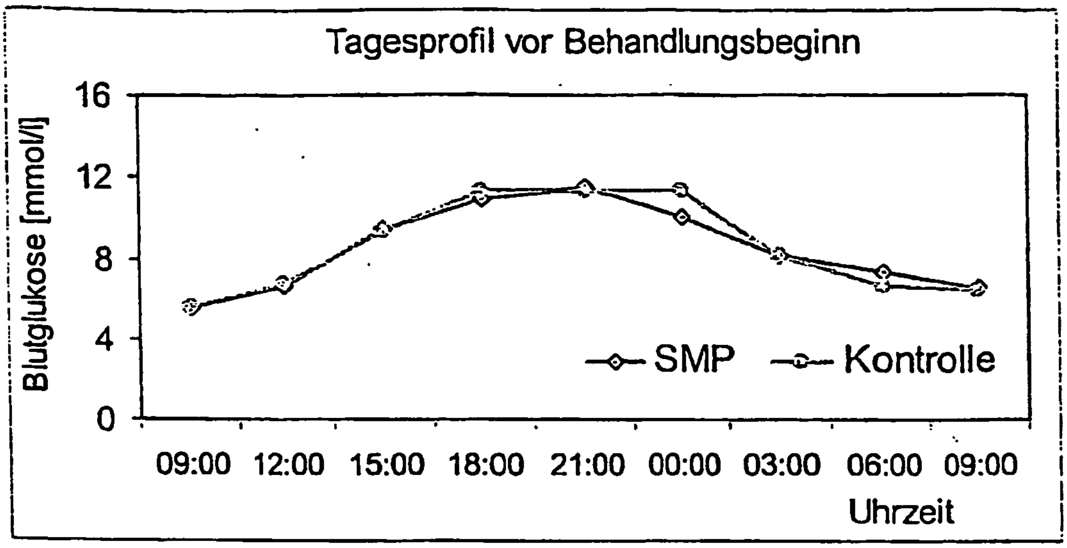


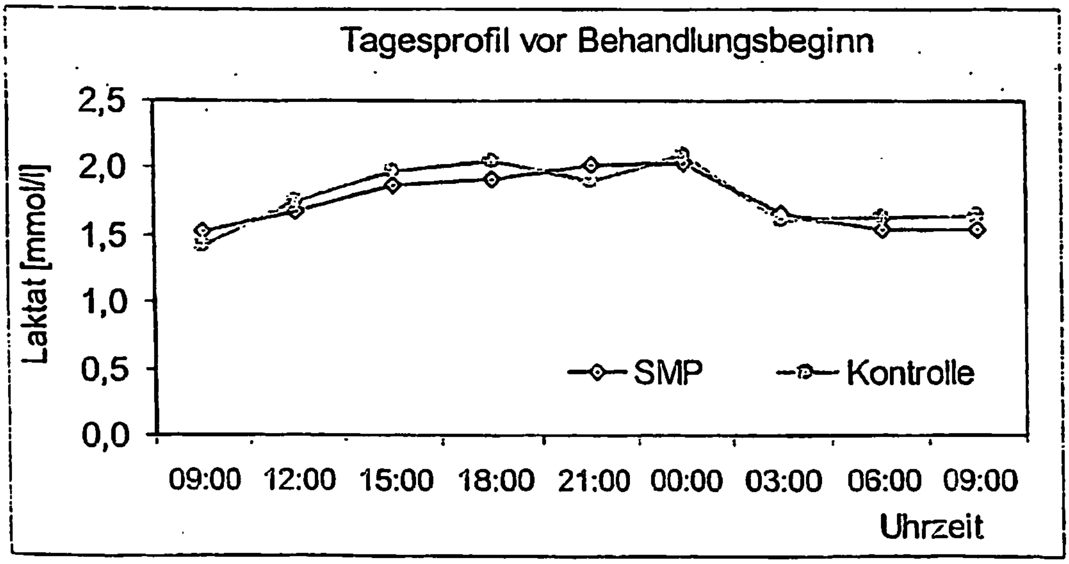

**Fig. 8 :** Blutglukose- und Laktat-Tagesprofil nach 6 Wochen Behandlung

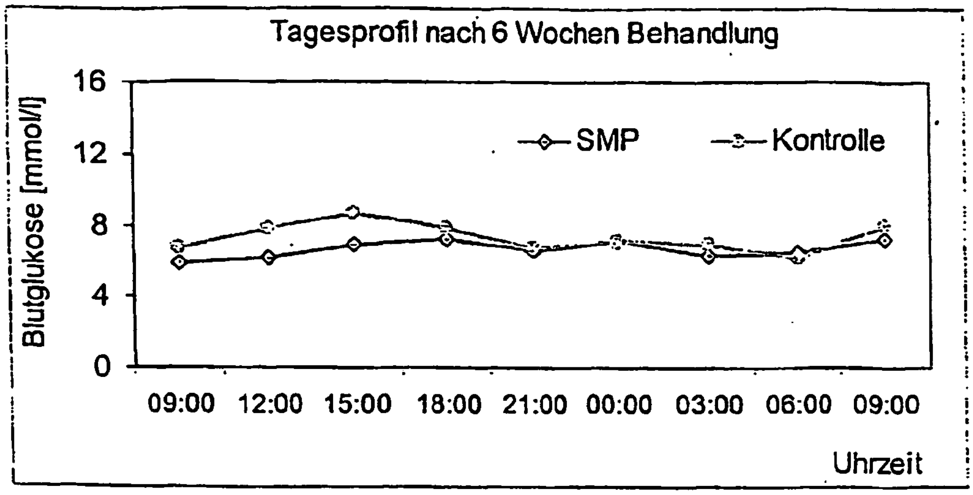


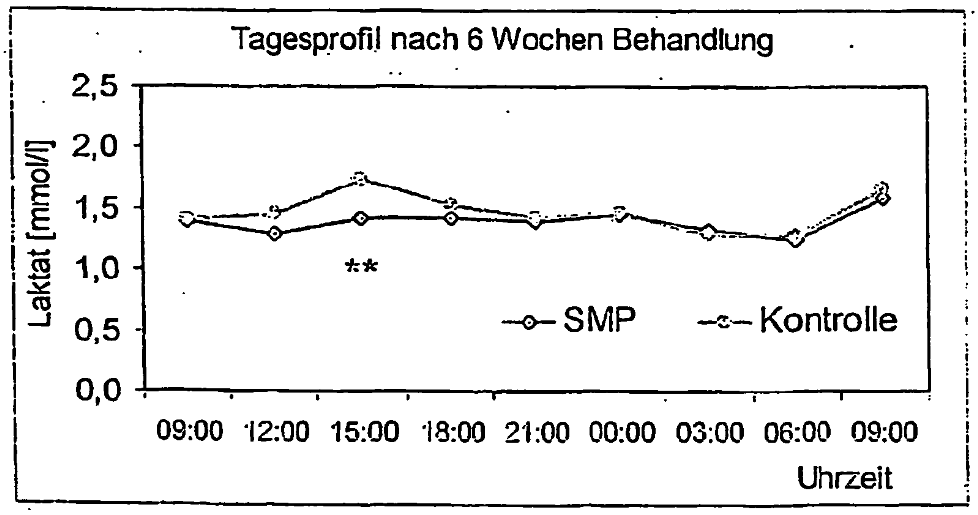


** p<0.01 vs. Kontrolle

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 20030004095 A
- DE 19854749 A1
- DE 10008880 A1
- DE 29623285 U1


### In der Beschreibung aufgeführte Nicht-Patentliteratur


- **Reaven GM.** *Curr. Opin. Lipidol.,* 1997, vol. 8 (1), 23-27
- **D. Tanne et al.** *Circulation,* 2001, vol. 104, 2892-2897
- *Diabety Care,* 2001, vol. 24, 1335-1341
- **Yoshioka.** *Metabolism,* 1993, vol. 42 (1), 75-80
- **P.Kovacs et al.** *Ann.N.Y.Acad.Sci.,* 1997, vol. 827, 94-99
- **P.Kovacs et al.** *Biochem.Biophsy.Res.Commun.,* 2000, 660-665
- **J.van den Brandt et al.** *Int.J.Obesity,* 2000, vol. 24, 1618-1622
- **J.van den Brandt et al.** *Metabolism,* 2000, vol. 49, 1140-1144